# EUROPEAN PATENT APPLICATION

(11) **EP 4 389 902 A1**
(43) Date of publication of application: **26.06.2024**
(21) Application number: 22892931.1
(22) Date of filing: 15.11.2022
(51) Int. Cl.: C12N 15/85, C12N 5/10

(54) **METHOD FOR PRODUCING HUMAN ARTIFICIAL CHROMOSOME VECTOR IN HUMAN CELLS**

(30) Priority: 15.11.2021 JP 2021185959
(71) Applicant: National University Corporation Tottori University, Tottori-shi, Tottori 680-8550 (JP)
(72) Inventor: KAZUKI Yasuhiro, Yonago-shi, Tottori 683-8503 (JP); UNO Narumi, Hachioji-shi, Tokyo 192-0392 (JP); TOMIZUKA Kazuma, Hachioji-shi, Tokyo 192-0392 (JP)
(74) Representative: D Young & Co LLP
(86) International application number: PCT/JP2022/042349
(87) International publication number: WO 2023/085433

(57) **Abstract**

Provided is a method for producing a human artificial chromosome vector with high safety by using a human cell. The method for producing a human cell comprising a human artificial chromosome vector comprises: substantially eliminating endogenous genes of long-arm and short-arm in a disomic human cell containing a pair of the homologous human chromosomes or a trisomic human cell containing trisomy of the homologous human chromosomes, from one of two chromosomes of the disomy or one or two of three chromosomes of the trisomy, thereby producing a cell population containing a human cell containing a human artificial chromosome vector comprising long-arm moiety and short-arm moiety substantially containing no endogenous gene, a human centromere, and a telomere; and collecting the human cell containing the human artificial chromosome vector from the cell population. As well as, the human cell comprises the human artificial chromosome vector.

## Description

### Technical Field

The present invention relates to a method for producing a human cell in which a human artificial chromosome vector that is originated from a human chromosome and substantially comprises no endogenous gene is formed in a human cell such as a normal or aneuploid human cell.

The present invention also relates to a human cell comprising the human artificial chromosome vector described above.

### Background Art

Conventional artificial chromosome vectors such as bacterial chromosome vectors (BAC) and yeast chromosome vectors (YAC) have a limitation in the sizes of transducing genes (or transgenes), and it is difficult to introduce chromosomes or fragments thereof with megabase (Mb) sizes into such vectors. Additionally, such vectors are integrated into host genomes, and thus loss of the transducing genes often occurs. As alternatives to such vectors, mouse artificial chromosome vectors and human artificial chromosome vectors have been developed by the present inventors, and for example, a mouse that can produce a human protein (for example, human antibody or the like) has been created (Patent Literature 1).

A human artificial chromosome vector comprises a human centromere, a long-arm fragment in the vicinity of the centromere (in which most of endogenous genes are eliminated), and a telomere, produced by modifying a human chromosome, and further comprises a site-directed recombinant enzyme recognition site into which an exogenous gene is inserted. Production of such a vector comprises fusing a human fibroblast with a mouse A9 cell, further introducing a human chromosome into a chicken DT40 cell that may be modified by homologous recombination, and further removing endogenous gene regions as many as possible in the DT40 cell to produce the human chromosome vector (Patent Literature 1, and Non Patent Literature 1 and 2).

Regarding the modification of chromosomes in human cells, Patent Literature 2 and 3 describe methods for site-directed DNA cleavage and homogenous integration in mammalian cells, such as human cells, using genome editing with CRISPR/Cas9.

Patent Literature 3 describes a method for producing a cell retaining a human artificial chromosome vector by using a micronucleate cell originated from a human cell. This method also comprises producing a human chromosome vector from a human cell via a mouse A9 cell, as well as a chicken DT40 cell as described in Patent Literature 1, and then introducing the vector into a Chinese hamster ovary (CHO) cell, as well as an induced pluripotent stem (iPS) cell by microcell-mediated chromosome transfer (MMCT).

However, research and development for enabling the use of human artificial chromosome vectors in advanced medicine, including regenerative medicine, gene therapy, and the like, have not been carried out to date.

### Citation List

### Patent Literature

Patent Literature 1: Japanese Patent Publication No. 2007-295860
Patent Literature 2: U.S. Patent No. 8895308
Patent Literature 3: WO2020/075823A1

### Non Patent Literature

Non Patent Literature 1: Katoh, M. et al., Biochem. Biophys. Res. Commun. 2004; 321 (2): 280-290
Non Patent Literature 2: Kazuki, Y. et al., Gene Ther. 2011; 18 (4): 384-393
Non Patent Literature 3: Cong, L. et al., Science 2013; 339 (6121): 819-823
Non Patent Literature 4: Kotini, A. G., Exp. Hematol. 2020; 87: 25-32

### Summary of Invention

### Technical Problem

In the prior art literature described above, it has been impossible to produce human artificial chromosome vectors in xeno-free (i.e., containing no component originated from animal other than human) environments by techniques in which safety is ensured at levels where clinical application to humans is possible. In addition, production of human chromosome vectors from which endogenous gene regions in human chromosomes are substantially or preferably entirely removed has not been described.

Furthermore, there have been no examples in which human artificial chromosome vectors have been used in regenerative medicine, and when such vectors are used, the challenge is to produce pharmaceuticals in highly safe and xeno-free environments. Accordingly, when human artificial chromosomes are to be produced by conventional methods, it would be considerably difficult to completely prevent contamination of foreign substances originated from mice and chickens upon constructing vectors as mouse cells and chicken cells being used.

In addition, diploid human cells have been considered unsuitable for use in the construction of human artificial chromosomes due to hetero-insufficiency caused by monosomy required in methods for producing human artificial chromosomes to date.

### Solution to Problem

The present inventors now found a method for producing a highly safe human artificial chromosome vector by using a human cell rather than a nonhuman animal cell.

The present invention encompasses the following features.
[1] A method for producing a human cell comprising a human artificial chromosome vector comprising: substantially eliminating endogenous genes of long-arm and short-arm in a disomic human cell containing a pair of the homologous human chromosomes or a trisomic human cell containing trisomy of the homologous human chromosomes, from one of two chromosomes of the disomy or one or two of three chromosomes of the trisomy, thereby producing a cell population containing a human cell containing a human artificial chromosome vector comprising long-arm moiety and short-arm moiety substantially containing no endogenous gene, a human centromere, and a telomere; and collecting the human cell containing the human artificial chromosome vector from the cell population.
[2] The method according to [1], wherein the collected human cell comprises two copies of a gene for haplo-insufficiency when the disomic human cell or the trisomic human cell comprises haplo-insufficiency.
[3] The method according to [1] or [2], further comprising inserting a sequence for site-directed DNA insertion into the human artificial chromosome vector.
[4] The method according to any of [1] to [3], further comprising inserting an exogenous gene or DNA into the human artificial chromosome vector.
[5] The method according to any of [1] to [4], wherein the trisomic human cell is a cell originated from a trisomic human patient or a trisomic cell artificially produced from a disomic cell.
[6] The method according to any of [1] to [5], wherein the human cell is a somatic cell, a progenitor cell, or a stem cell.
[7] A human cell comprising a pair of homologous human chromosomes and one human artificial chromosome vector originated from a human chromosome homogenous thereto, wherein the human artificial chromosome vector comprises long-arm moiety and short-arm moiety containing substantially no endogenous gene, a human centromere, and a telomere.
[8] A human cell comprising one human chromosome and one or two human artificial chromosome vectors originated from a human chromosome homogenous thereto, wherein the human artificial chromosome vector comprises long-arm moiety and short-arm moiety substantially containing no endogenous gene, a human centromere, and a telomere, and the cell comprises two copies of a gene for haplo-insufficiency.
[9] The human cell according to [7] or [8], wherein the human cell is a human cell produced by the method according to any of [1] to [6].
[10] The human cell according to any of [7] to [9], wherein the human artificial chromosome vector comprises a sequence for site-directed DNA insertion.
[11] The human cell according to any of [7] to [10], wherein the human artificial chromosome vector comprises an exogenous gene or DNA.
[12] The human cell according to any of [7] to [11], wherein the human cell is a somatic cell, a progenitor cell, or a stem cell.
[13] The human cell according to [12], wherein the stem cell is an iPS cell or mesenchymal stem cell.

The present specification encompasses the contents disclosed in Japanese Patent Application No. 2021-185959 on which the priority of the present application is based.

### Advantageous Effects of Invention

The present invention enables a human artificial chromosome vector to be introduced into human cells (including human iPS cells) without using or stepping through a nonhuman cell, and therefore, enables production of a human cell comprising a human artificial chromosome vector without nonhuman animal cell component.

### Brief Description of Drawings

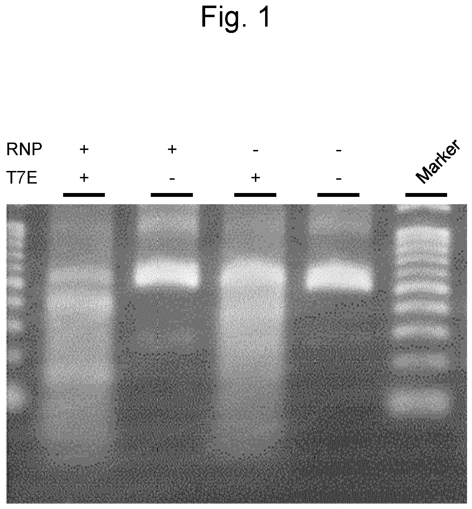

[Figure 1] This figure shows the results of the evaluation of the cleavage activity of telomere guide RNA (gRNA) for eliminating the entire gene region on the long-arm of human chromosome 21. In the figure, lane 1 shows the result of adding T7E to the PCR product of DNA originated from a ribonucleoprotein complex ("RNP", which is composed of CRISPR-Cas9 protein)-introduced cell. Lane 2 shows the result of adding no T7E to the PCR product of DNA originated from an RNP-introduced cell. Lane 3 shows the result of adding T7E to the PCR product of DNA originated from an RNP-unintroduced cell. Lane 4 shows the result of adding no T7E to the PCR product of DNA originated from an RNP-unintroduced cell. In addition, lane 5 shows a DNA size index marker ("Marker" in the figure), which is Gene ladder wide 2. The results are from Example 2.

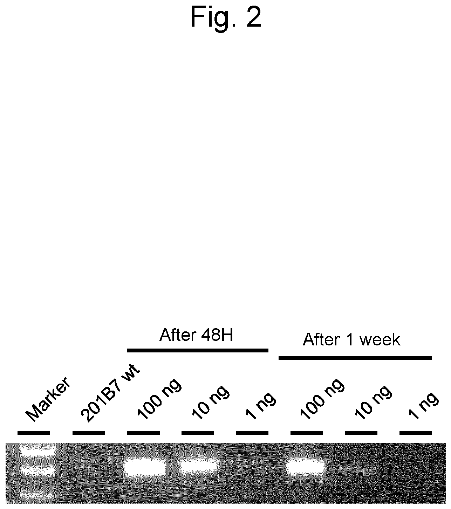

[Figure 2] This figure shows the results of the analysis of a PCR amplified product obtained by introducing guide RNA (gRNA) into a human iPS cell strain (201B7) in order to eliminate the long-arm of human chromosome 21. Lane 1 is a DNA ladder marker, which is Gene ladder wide 2. Lane 2 shows the result of PCR using DNA ("201B7 wt" in the figure) originated from an untreated wild type 201B7 cell. Lanes 3 to 8 show the results of analysis using genomic DNAs collected 48 hours and 1 week after the introduction of a combination of gRNAs for eliminating the long-arm of chromosome 21 into 201B7. It is shown that amplified products specific for elimination of the long-arm of chromosome 21 were detected in the case of using samples each containing around 1500 cells equivalent to 10 ng of DNA even when the genomic DNAs collected after 1 week were used. In the figure, "100 ng", "10 ng", and "1 ng" show the amounts of template DNAs used, respectively. The results are from Example 3.

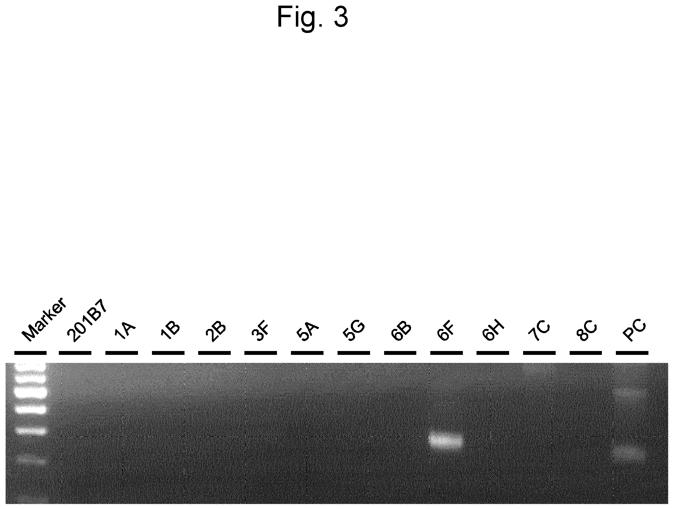

[Figure 3] This figure shows the results of determining elimination of a long-arm region in a cell population, in which the long-arm of human chromosome 21 was eliminated, for an amplified product from a PCR method. Lane 1 shows a DNA size index marker. It is shown that no amplified product specific for elimination of the long-arm of a chromosome was obtained for DNAs originated from positions other than 6F shown in lanes 2 to 9, and 11 to 13, while an amplified product of interest (245 bp) was obtained in lane 10 for a DNA originated from position 6F. PC shows a positive control. The results are from Example 4.

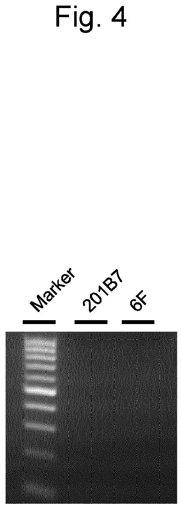

[Figure 4] This figure shows that no amplified product specific for occurrence of inversion of the long-arm of chromosome 21 was detected when a DNA originated from position 6F was used in determination of elimination of a long-arm region in a cell population in which the long-arm of human chromosome 21 was eliminated. The results are from Example 4.

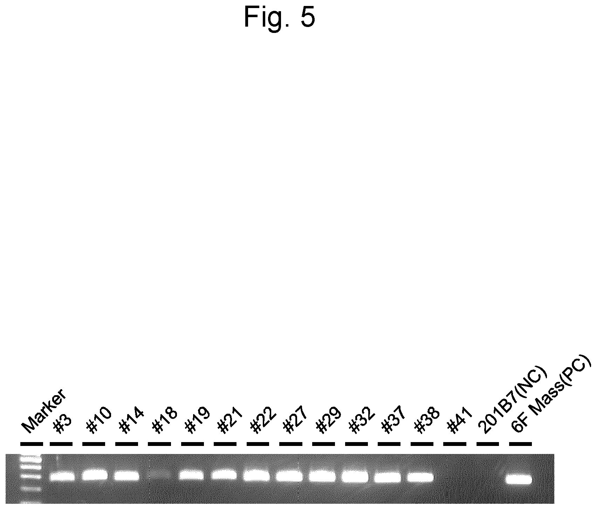

[Figure 5] This figure shows the results of confirmation of amplified products of interest (245 bp) in 12 subclones (#3, #10, #14, #18, #19, #21, #22, #27, #29, #32, #37, and #38) out of 49 subclones by a PCR method for the cell population of position 6F in which the full length of the long-arm of chromosome 21 was eliminated. In the figure, "201B7" shows the result for using an untreated 201B7 cell, NC shows a negative control, and PC shows a positive control. The results are from Example 5.

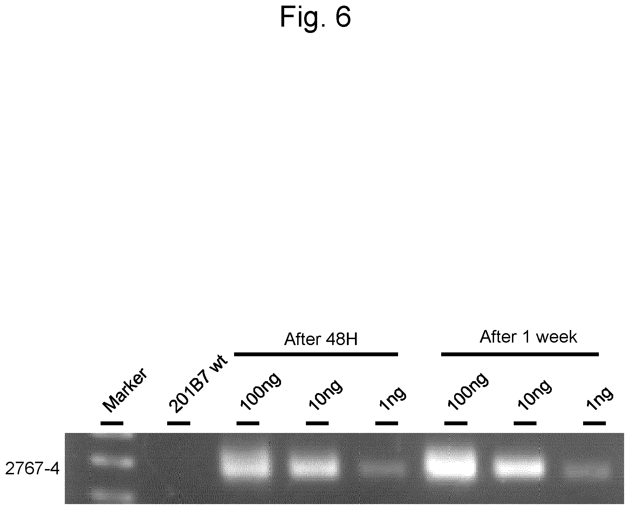

[Figure 6] This figure shows the results of confirmation of an amplified product specific for elimination of the long-arm of chromosome 21 in a human trisomy 21 iPS cell strain (2767-4) by a PCR method. The results of analysis using genomic DNAs collected 48 hours and 1 week after the introduction of a combination of gRNAs for eliminating the long-arm of chromosome 21 is shown. It is shown that amplified products specific for elimination of the long-arm of chromosome 21 were detected in the case of using samples each containing around 150 cells equivalent to 1 ng of DNA even when the genomic DNAs collected after 1 week were used. In the figure, "201B7 wt" shows use of DNA originated from an untreated disomic 201B7 cell. The results are from Example 6.

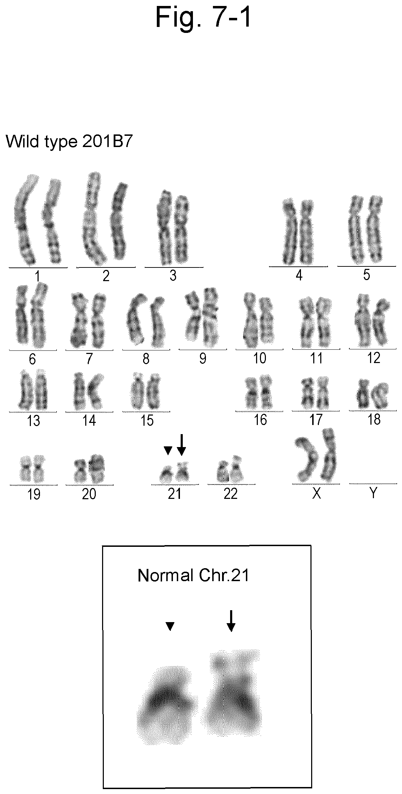

[Figure 7-1] This figure shows the results of confirmation of the presence of a chromosome indicating no elimination of long-arm 21 in a human iPS cell in the case of using a disomic strain (201B7) by a G-Band method. The results are from Example 7.

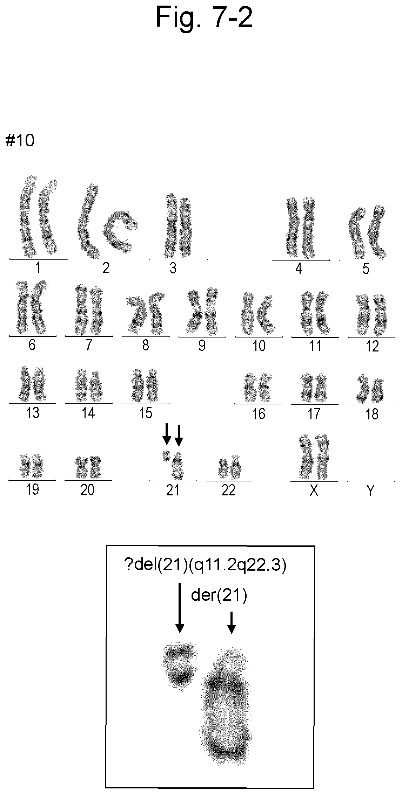

[Figure 7-2] This figure shows the results of confirmation of the presence of a chromosome fragment indicating elimination of the long-arm of chromosome 21 (?del (21) (q11.2q22.3)) in a human iPS cell in the case of using #10 clone by a G-Band method. In the #10 clone, the chromosome fragment indicating the elimination of the long-arm of chromosome 21 was observed in comparison with a wild type 201B7 cell. The results are from Example 7.

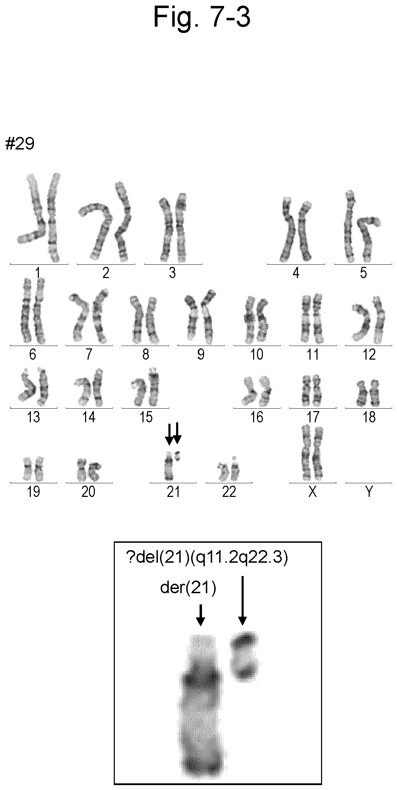

[Figure 7-3] This figure shows the results of confirmation of the presence of a chromosome fragment indicating elimination of the long-arm of chromosome 21 (?del (21) (q11.2q22.3)) in a human iPS cell in the case of using #29 clone by a G-Band method. In the #29 clone, the chromosome fragment indicating the elimination of the long-arm of chromosome 21 was observed in comparison with wild type 201B7. The results are from Example 7.

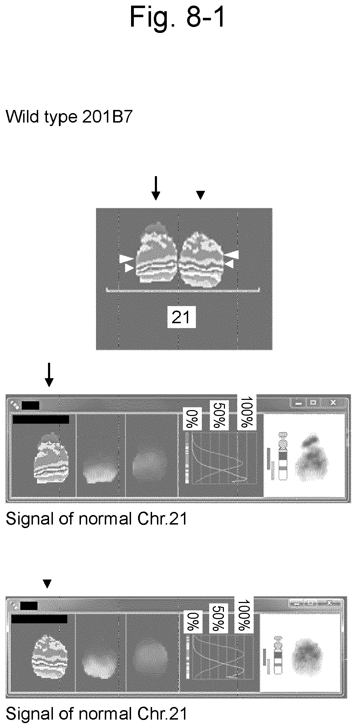

[Figure 8-1] This figure shows the results of confirmation of the presence of chromosome 21, of which the long-arm is not eliminated, in a human iPS cell in the case of using a disomic strain (201B7) by an mBand method. In the figure, a white arrow head (large) shows the stain of the upper half of the long-arm (q11.2 to q22.1) of the chromosome 21 (pink color), and a white arrow head (small) shows the stain of the lower half of the long-arm (q22.1 to q22.3) of the chromosome 21 (green). In the figure, a black arrow shows one homologous chromosome of chromosome 21, and a black arrow head shows the other homologous chromosome of chromosome 21. The results are from Example 8.

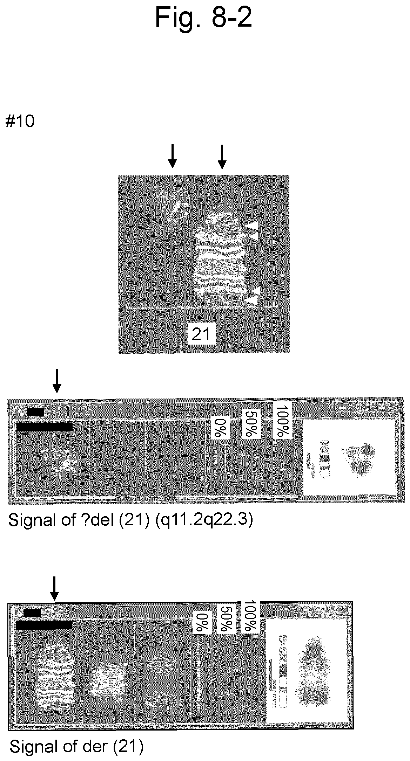

[Figure 8-2] This figure shows the results of confirmation of the presence of a chromosome fragment indicating elimination the long-arm of chromosome 21 in a human iPS cell in the case of using #10 clone by an mBand method. In the figure, a white arrow head (large) and a white arrow head (small) are the same as those described in Figure 8-1. In the #10 clone, a chromosome fragment indicating elimination of the long-arm of chromosome 21 was observed in comparison with wild type 201B7 (Figure 8-1). The results are from Example 8.

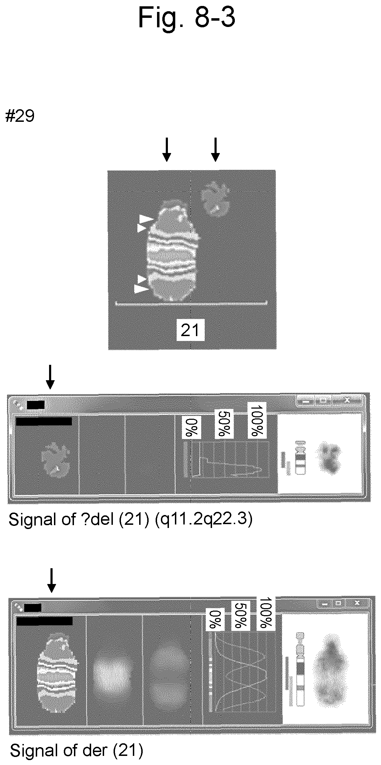

[Figure 8-3] This figure shows the results of confirmation of the presence of a chromosome fragment indicating elimination the long-arm of chromosome 21 in a human iPS cell in the case of using #29 clone by an mBand method. In the figure, a white arrow head (large) and a white arrow head (small) are the same as those described in Figure 8-1. In the #29 clone, a chromosome fragment indicating elimination of the long-arm of chromosome 21 was observed in comparison with wild type 201B7 (Figure 8-1). The results are from Example 8.

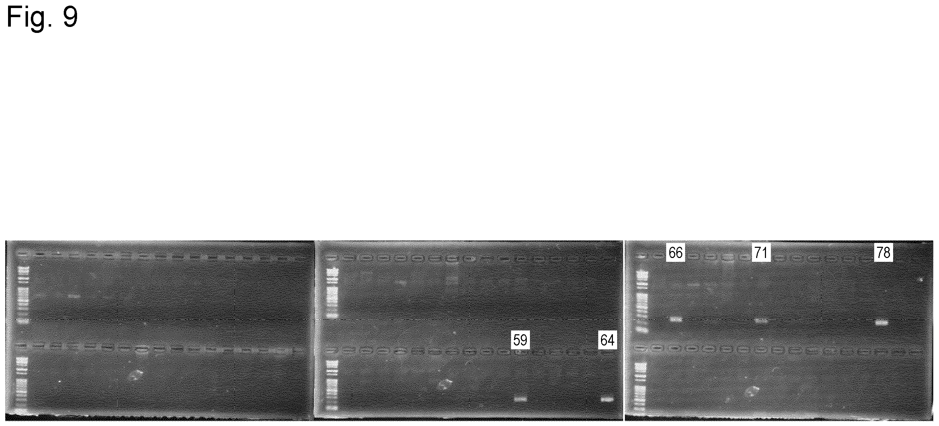

[Figure 9] This figure shows the results of determining elimination of a long-arm region in a cell population, in which the long-arm of human chromosome 21 was eliminated, in a human 21 trisomic iPS cell strain for an amplified product from a PCR method. It is shown that amplified products specific for chromosome with the long-arm being eliminated were obtained for DNAs originated from 96-well Nos. 59, 64, 66, 71, and 78 positions. The results are from Example 9.

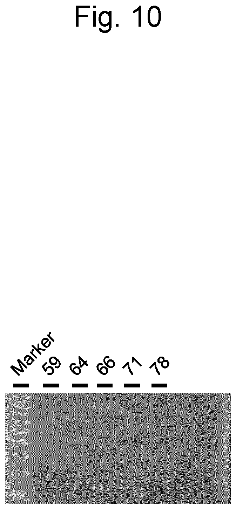

[Figure 10] This figure shows the results of determining elimination of a long-arm region in a cell population, in which the long-arm of human chromosome 21 was eliminated, in a human 21 trisomic iPS cell strain for an amplified product from a PCR method. It is shown that amplified products specific for chromosome long inversion were not obtained for DNAs originated from 96-well Nos. 59, 64, 66, 71, and 78 positions.

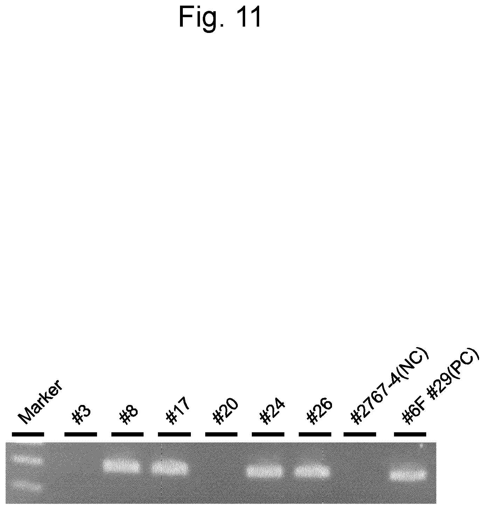

[Figure 11] This figure shows that an amplified product of interest (245 bp) was confirmed in 4 subclones (#8, 17, 24, and 26) out of 26 subclones for a cell population in which the long-arm of human chromosome 21 was eliminated. The results are from Example 9.

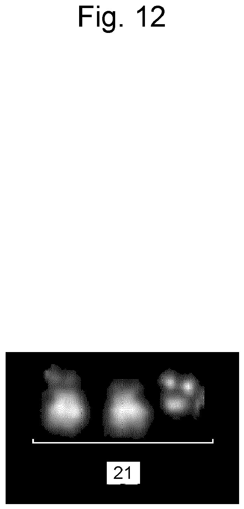

[Figure 12] This figure shows the results of confirmation of the presence of a chromosome fragment indicating elimination of the long-arm of chromosome 21 in a human iPS cell in the case of using a trisomic strain (2767-4) by a DAPI staining method. In a #26 clone, a stained fragment indicating elimination of the long-arm of chromosome 21 (right end in the figure) was observed in comparison with normal chromosome 21 (left end and center in the figure). The results are from Example 10.

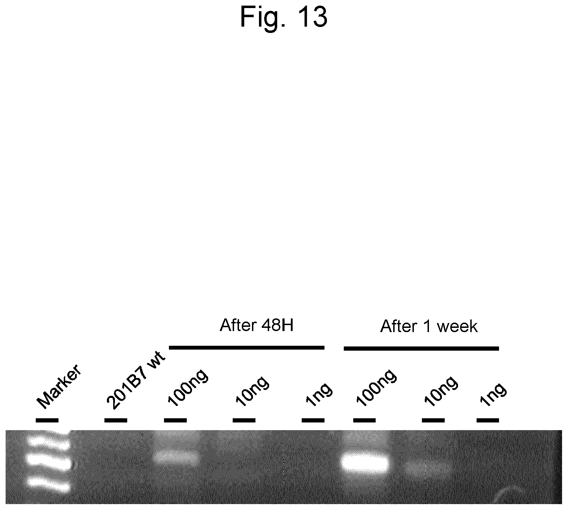

[Figure 13] This figure shows the results of analysis using genomic DNAs collected 48 hours and 1 week after the introduction of a combination of gRNAs for eliminating the short arm of chromosome 13 into a normal karyotype iPS cell (201B7). It is shown that amplified products specific for elimination of the short-arm of chromosome 13 were detected in the case of using samples each containing around 1500 cells equivalent to 10 ng of DNA even when the genomic DNAs collected after 1 week were used. The results are from Example 11.

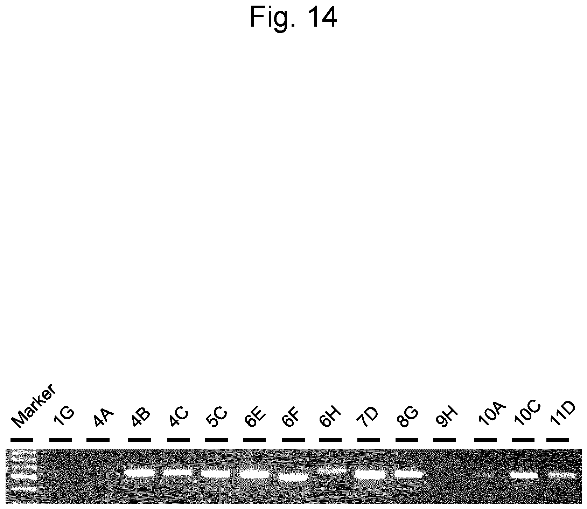

[Figure 14] This figure shows the results of determining elimination of a short-arm region in a cell population, in which the long-arm of human chromosome 13 was eliminated, in a normal karyotype iPS cell strain (201B7) for an amplified product from a PCR method. It is shown that amplified products specific for human chromosome 13 with the short-arm being eliminated were obtained for DNAs (up to 4B to 8G, 10A, 10C, and 11D) originated from 11 positions including 96-well Nos. 4C and 5C positions. The results are from Example 12.

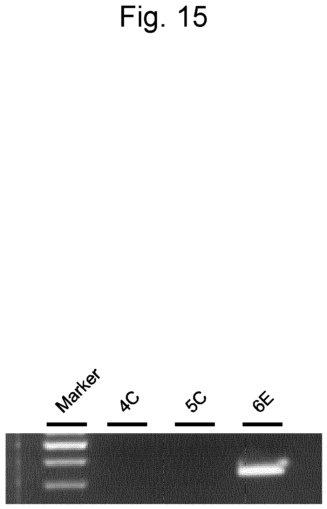

[Figure 15] This figure shows the results of determining elimination of a short-arm region in a cell population, in which the short-arm of human chromosome 13 was eliminated, in a normal karyotype iPS cell strain (201B7) for an amplified product from a PCR method. It is shown that amplified products specific for chromosome short-arm inversion were not obtained for DNAs originated from 96-well Nos. 4C and 5C positions. The results are from Example 12.

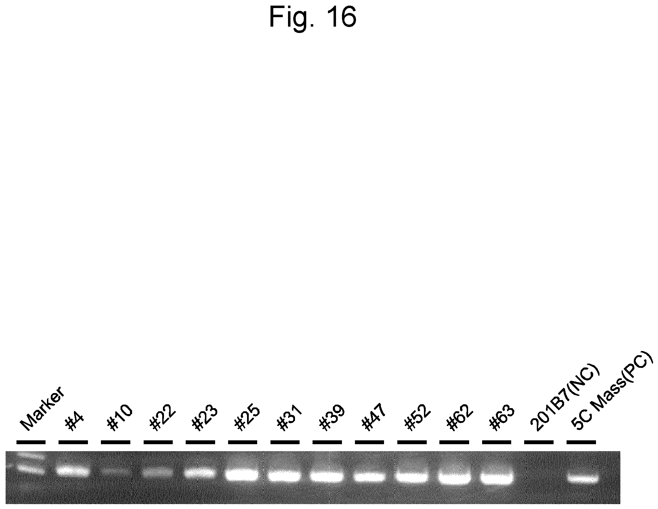

[Figure 16] This figure shows that amplified products specific for chromosome with the short-arm being eliminated were obtained for DNAs originated from 96-well Nos. #4, 10, 22, 23, 25, 31, 39, 47, 52, 62, and 63 positions in a subclone, in which the long-arm of human chromosome 13 was eliminated, in a normal karyotype iPS cell strain (201B7). The results are from Example 13.

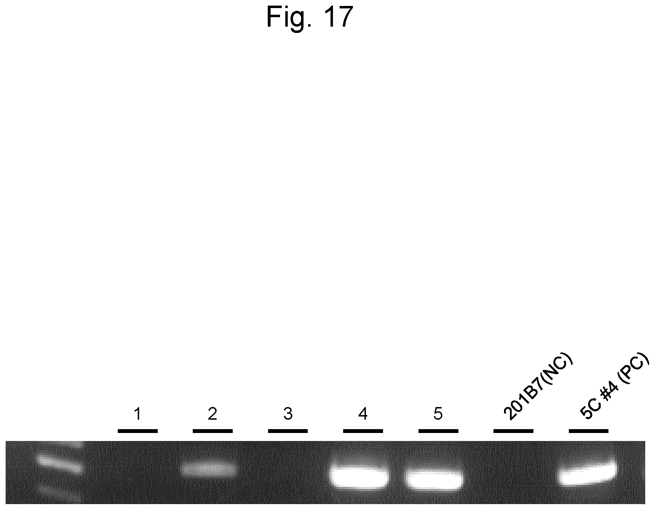

[Figure 17] This figure shows that amplified products specific for chromosome with the short-arm being eliminated were obtained for DNAs originated from subclones #2, 4, and 5 originated from a 96-well No. #4 position in a subclone, in which the long-arm of human chromosome 13 was eliminated, in a normal karyotype iPS cell strain (201B7). The results are from Example 13.

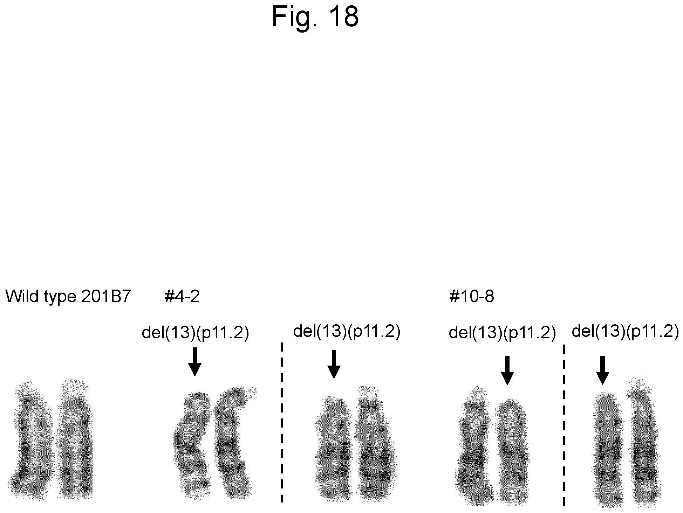

[Figure 18] This figure shows the results of confirmation of the presence of a chromosome fragment and chromosome indicating elimination of No. 13 short-arm in the case of using a disomic strain (201B7) in a human iPS cell by a G-Band method. In subclones #4-2 (subclone #2 of subclone #4 from cell population 5C) and # 10-8 (subclone #8 of subclone #10 from cell population 5C), a chromosome fragment indicating elimination of the short-arm of chromosome 13 (del (13) (p11.2), shown by arrow) was shown to be observed in comparison with normal chromosome 13 ("wild type 201B7" in the figure). The results are from Example 14.

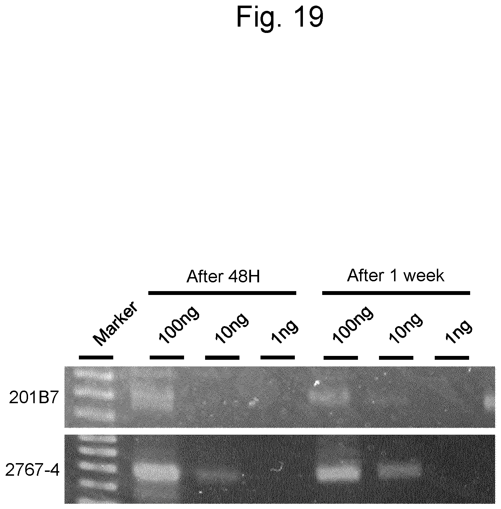

[Figure 19] This figure shows the results of confirmation of the presence of an amplified product specific for chromosome 21 with the short-arm being eliminated in an iPS cell strain into which guide RNA (gRNA) was introduced in order to eliminate the short-arm of human chromosome 21 by a PCR method. Lanes 2 to 7 show the results of analysis using genomic DNAs collected 48 hours and 1 week after the introduction of a combination of gRNAs for eliminating the short-arm of chromosome 21. Lane 1 shows a DNA ladder marker ("Marker" in the figure). In the figure, "100 ng", "10 ng", and "1 ng" show the amounts of template DNAs used, respectively. The upper section shows the results in the case of using a 201B7 cell ("201B7" in the figure) which was a disomic human iPS cell strain, and the lower section shows the results in the case of a 2767-4 cell ("2767-4" in the figure) which was a trisomic human iPS cell strain. The efficiency of eliminating the short-arm of chromosome 21 was found to be increased by using the trisomic cell strain.

### Description of Embodiments

The present invention is described in more detail.

### 1. Method for Producing Human Cell Comprising Human Artificial Chromosome Vector

Provided in a first aspect according to the present invention is a method for producing a human cell comprising a human artificial chromosome vector comprising: substantially eliminating endogenous genes of long-arm and short-arm in a disomic human cell containing a pair of the homologous human chromosomes or a trisomic human cell containing trisomy of the homologous human chromosomes, from one of two chromosomes of the disomy or one or two of three chromosomes of the trisomy, thereby producing a cell population containing a human cell containing a human artificial chromosome vector comprising long-arm moiety and short-arm moiety substantially containing no endogenous gene, a human centromere, and a telomere; and collecting the human cell containing the human artificial chromosome vector from the cell population.

The human chromosome that may be used in the method described above may be any of human chromosomes 1 to 22 and sex chromosomes (X and Y), and the trisomy described above is the trisomy of any of human chromosomes 1 to 22 and sex chromosomes.

The term "disomy" as used herein mean a pair of homogenous human chromosomes, and may be a pair of normal chromosomes, or a pair of human chromosomes comprising a gene involved in a phenomenon in which a mutation in one of allelic genes causes the amount of protein produced from the genes to be insufficient, resulting in dysfunction, so-called haplo-insufficiency (referred to as "gene for haplo-insufficiency").

The term "trisomy" as used herein means a human cell showing aneuploid chromosome aberration further comprising an extra homogenous chromosome in addition to a pair of homologous chromosomes. In humans, for example, trisomy 21, trisomy 19, trisomy 18, trisomy 16, trisomy 13, trisomy 7, and the like are known as such chromosome aberration, and, for example, in trisomy 21, three homogenous chromosomes 21 are contained in a human cell.

The method of the present invention is characterized by comprising producing a human artificial chromosome vector from one of the two disomic chromosomes comprising a pair of homologous human chromosomes of a subject (or of interest), or from one or two of three trisomic chromosomes of a subject (or of interest), in a human cell. The vector comprises a human centromere and telomere, but the long-arm and the short-arm thereof do not substantially comprise an endogenous gene. The term "substantially" herein means that one or plural genes, for example, 30 or less, 20 or less, 10 or less, 5 or less, or 2 or less genes, excluding a causative gene causing chromosome aberration when the human cell of the present invention is used in a therapy or the like, may be contained. In the present invention, however, in consideration of therapeutic safety, it is preferable that the above-described vector does not comprise all the endogenous genes of the long-arm and the short-arm.

### [Production of Human Artificial Chromosome Vector from Human Cell]

### <Disomic or Trisomic Human Cell>

The disomic human cell may be a cell comprising a pair of homologous chromosomes of a subject (or of interest), or may be a human cell comprising a gene for haplo-insufficiency, as described above.

Such trisomic human cells include, for example, a cell strain originated from a trisomic human patient as described above, an induced pluripotent stem (iPS) cell produced from a cell originated from a trisomic human patient, an artificial trisomic human cell artificially produced by introducing one of human chromosomes into a normal human cell, and the like. Such a human cell may optionally comprise a gene for haplo-insufficiency.

Such cells originated from trisomic human patients as described above include, without limitation, for example, fibroblast cell strains from patients with Down's syndrome, for example, JCRB9044 Detroit 532 (originated from skin), ATCC CCL-54^{™} Detroit 532 (originated from skin), CORIELL Institute GM02767 (originated from fibroblast), and the like.

Such iPS cells produced from such cells originated from trisomic human patients as described above include, without limitation, for example, iPS cells originated from fibroblasts from patients with Down's syndrome, for example, ATCC-DYP0730 IPS Cells (ACS-1003^{™}) (the cells were produced by reprogramming (initiating) ATCC CCL-54^{™} Detroit 532 (human Down's syndrome cell strain) under the expression of Oct4, Sox2, Klf4, and Myc genes), SKIP001644: ND50026 (SKiP Stemcell Search), and the like.

Herein, iPS cells may be produced by the following known techniques. Specifically, such iPS cells as described above form a colony about 3 to 5 weeks after introducing certain reprogramming factors (DNAs or proteins) into somatic cells (including somatic stem cells), culturing the cells in an appropriate medium, and further subculturing the cells. As the reprogramming factors, for example, a combination consisting of Oct3/4, Sox2, Klf4, and c-Myc; a combination consisting of Oct3/4, Sox2, and Klf4; a combination consisting of Oct4, Sox2, Nanog, and Lin28; and a combination consisting of Oct3/4, Sox2, Klf4, c-Myc, Nanog, and Lin28 are known (K. Takahashi and S. Yamanaka, Cell; 126: 663-676 (2006); WO2007/069666; M. Nakagawa et al., Nat. Biotechnol. 26: 101-106 (2008); K. Takahashi et al., Cell, 131: 861-872 (2007); J. Yu et al., Science; 318: 1917-1920 (2007); J. Liao et al., Cell; Res. 18, 600-603 (2008)).

The culture comprises using, for example, a mitomycin C-treated mouse embryo fibroblast strain (for example, STO) as a feeder cell, and culturing a reprogramming factor expression vector-introduced somatic cell (about 10⁴ to 10⁵ cells/cm²) on the feeder cell layer using a medium for an ES cell at a temperature of about 37°C. In this case, the feeder cell is not indispensable (Takahashi, K. et al., Cell; 131: 861-872 (2007)). The basal medium is, for example, a Dulbecco's modified Eagle's medium (DMEM), an Eagle's minimum essential medium (EMEM), a Ham's F-12 medium, an RPMI-1640 medium, an Iscove's modified Dulbecco's medium (IMDM), or a mixed medium thereof. A medium for a primate ES cell (REPROCELL Inc. Japan) or the like may be used as the medium for a human iPS cell. The feeder cell is not indispensable.

A method described in, for example, H. Nawata et al. (PLoS ONE 2011; 6 (9): e25319; Dysregulation of Gene Expression in the Artificial Human Trisomy Cells of Chromosome 8 Associated with Transformed Cell Phenotypes), K. Hiramatsu et al. (Biochem Biophys Res Commun. 2019 Jan 8; 508 (2): 603-607. Generation of a novel isogenic trisomy panel in human embryonic stem cells via microcell-mediated chromosome transfer), or the like may be used for producing the artificial trisomic human cell described above. The method of Nawata et al. comprises colcemid-treating and inducing a mouse A9 cell comprising an individual human chromosome, and then fusing a human chromosome-containing micronucleate cell, purified by a method such as centrifugation, with a human cell using microcell-mediated chromosome transfer (MMCT) to introduce the human chromosome into the human cell. In the method of the present invention, it is important to avoid the contamination of a cell component originated from a nonhuman animal, and therefore, it is preferable to use a method developed by the present inventors, that is, a method of culturing a human cell in a medium containing a micronucleation inducing agent (for example, microtubular polymerization inhibitor) to produce a micronucleate cell originated from a human cell (WO2020/075823) instead of the method for producing a micronucleate cell by the method of Nawata et al. By the method, a micronucleate cell comprising any of human chromosomes may be produced, and fused with a human cell using an MMCT method same as in the method of Nawata et al. to produce a trisomic human cell. The trisomic human cell may be identified by, for example, karyotype analysis.

### <Production of Human Artificial Chromosome Vector>

A method for producing a human artificial chromosome vector from the disomic or trisomic human cell described above is described.

In the method of the present invention, for example, for one of chromosomes in a disomic human cell, or one or two of three homologous chromosomes forming trisomy in a trisomic human cell, the endogenous genes of a long-arm and a short-arm are substantially eliminated using a technique such as genome editing (for example, CRISPR/Cas system), a telomere cleavage method, or a recombinant method by the system of a site-directed recombinant enzyme/recognition site for the enzyme, and optionally, a sequence for site-directed DNA insertion for loading an exogenous gene (for example, site-directed recombinant enzyme recognition sequence), and further optionally, an exogenous gene or a DNA may be inserted.

Regarding a site recognized by the site-directed recombinant enzyme described above, a phenomenon in which a certain enzyme recognizes a certain recognition site to specifically cause the recombination of DNA in the recognition site is known. In the human artificial chromosome vector in the present invention, an exogenous gene or DNA of interest may be inserted using a system consisting of such an enzyme and a recognition site for the enzyme. Examples of such a system may include a system of Cre enzyme originated from bacteriophage P1 and a loxP sequence as a recognition site therefor (Cre/loxP system; B. Sauer in Methods of Enzymology; 1993, 225: 890-900), a system of Flp enzyme originated from budding yeast and an FRT (Flp recombination target) sequence as a recognition site therefor (Flp/FRT system), a system of ϕ C31 integrase originated from *Streptomyces* phage and a ϕ C31 attB/attP sequence as a recognition site therefor, a system of R4 integrase and an R4 attB/attP sequence as a recognition site therefor, a system of TP901-1 integrase and a TP901-1 attB/attP sequence as a recognition site therefor, and a system of Bxb1 integrase and a Bxb1 attB/attP sequence as a recognition site therefor, but is not limited to the systems described above as long as the system can function as a DNA sequence insertion site. A known method, for example, a gene targeting or homologous recombination method, genome editing, or the like may be utilized for inserting such a recognition site for a site-directed recombinant enzyme. In the genome editing, using, for example, a CRISPR/Cas9 system as described below, together with a donor vector comprising the sequence of an exogenous gene or DNA, enables knock-in of the sequence of the exogenous gene or DNA into a target site.

The exogenous gene or DNA described above may comprise a human gene or DNA useful for therapy of, for example, the diseases, disorders, and the like of humans, and optionally reporter genes (for example, genes encoding fluorescent proteins, such as GFP, EGFP, and YFP), selectable marker genes (for example, drug resistant genes such as Neo^{R}, Amp^{R}, and BS^{R}), and the like. Examples thereof are any human exogenous genes or DNAs that can be used in regenerative medicine and gene therapy promising for therapy of chromosome aberration and the inherited diseases.

The term "human cell" as used herein encompasses, for example, somatic cells, progenitor cells, stem cells, and cells originated therefrom, and the types of the cells include cultured cells, established cells, passaged cells, artificially induced cells (for example, iPS cells), and the like. The kinds of the cells are not limited, and examples thereof include skin cells, lung cells, renal cells, hepatic cells, intestinal cells, renal cells, muscle cells, nerve cells, and myeloid cells. Examples of the human stem cells are tissue stem cells (that have self-renewal capacity and may be differentiated into cells in tissues), somatic stem cells (that may be differentiated into cells in the living body; for example, mesenchymal stem cells), and the like. Preferred examples of the cells are progenitor cells, iPS cells, stem cells such as mesenchymal stem cells, somatic cells originated from humans and the like. The mesenchymal stem cells may be obtained from the bone marrow, the umbilical blood, the adipose tissue, and the like.

A method for converting one or two of chromosomes in a disomic or trisomic human cell into a human artificial chromosome vector by genome editing is described below.

The genome editing is a technique to edit genomic DNA or perform genetic modification using, for example, an artificial cleaving enzyme such as TALEN (transcription activator-like effector nuclease) or ZFN (zinc finger nuclease), a CRISPR-Cas system, or the like. In the method of the present invention, any technique for genome editing may be used, and a CRISPR-Cas system is preferably used.

Among the systems described above, particularly the CRISPR/Cas9 system was discovered in the adaptive immunity mechanisms of bacteria and archaebacteria against viruses and plasmids. In the CRISPR/Cas9 system, the construction of a vector is relatively simple and easy, and a plurality of genes may be simultaneously modified (Jinek et al., Science, 17, 337 (6096): 816-821, 2012, Sander et al., Nature biotechnology, 32 (4): 347-355, 2014). The system comprises a Cas9 nuclease for cleaving double-stranded DNA on the genome and a guide RNA recognizing a target sequence on the genome and having a sequence with about 20 bp (sgRNA, or crRNA (CRISPR RNA) + tracrRNA (trans-activating CRISPR RNA)). Coexpression of them in a cell allows the gRNA to recognize a PAM sequence in the vicinity of the target sequence and to be specifically bound to target genomic DNA, and allows a Cas9 protein to induce double-strand break (DSB) upstream of 5' of the PAM sequence (NGG (N = C, G, A, or T)). The Cas9 protein that is currently most used is an SpCas9 type, and the position for introducing a mutation is less restricted because the PAM sequence is NGG. In the present invention, the sites on the long-arm and short-arm of a human chromosome targeted by the guide RNA are sites enabling elimination of an endogenous gene region.

Such sites are, for example, in the case of the long-arm of human chromosome 21, for example, sites with which the regions of NC_000021.9, homo sapiens chromosome 21, GRCh38.p13 primary assembly: nucleotide Nos. 13,084,315-46,670,306 can be eliminated, and in the case of the short-arm, sites with which the regions of NC_000021.9, homo sapiens chromosome 21, GRCh38.p13 primary assembly: nucleotide Nos. 5,011,894-10,540,501 can be eliminated; that is, each of sites 13,084,315, and 46,670,306 for the long-arm, and each of sites 5,011,894, and 10,540,501 for the short-arm.

Guide RNA sequences for cleaving the long-arm of human chromosome 21 and the short-arm of human chromosome 13, and target sequences thereof are, without limitation, for example, the following sequences. With regard to the sequences, thymine (T) is uracil (U) in the case of RNA. The target sequence is a nucleotide sequence to which a PAM sequence indicated at the 3' end of each sequence of SEQ ID NOs:1 to 4 is bound. The PAM sequence is added in a cell.

In the case of the long-arm of human chromosome 21:
gRNA (in the vicinity of Cen): 5'-GACAGGTAAGGATTACTAGG-3' (SEQ ID NO:1) + (AGG)-PAM
gRNA (in the vicinity of Tel): 5'-TAAAGATGTGCTCCTTCCCG-3' (SEQ ID NO:2) + (AGG)-PAM

In the case of the short-arm of human chromosome 13:
gRNA (in the vicinity of Cen): 5'-AGAACTGAAGCATCACTCAC-3' (SEQ ID NO:3) + (TGG)-PAM
gRNA (in the vicinity of Tel): 5'-AGAAGCGAATCCTGCTTGCA-3' (SEQ ID NO:4) + (GGG)-PAM

Guide RNA sequences for cleaving the short-arm of human chromosome 21 and target sequences thereof are, without limitation, for example, the following sequences. With regard to the sequences, thymine (T) is uracil (U) in the case of RNA. The target sequence is a nucleotide sequence to which a PAM sequence indicated at the 3' end of each sequence of SEQ ID NOs:15 and 16 is bound.

In the case of the long-arm of human chromosome 21:
gRNA (in the vicinity of Tel): 5'-AGACTGTAGCCTTGACCCAG-3' (SEQ ID NO:15) + (AGG)-PAM
gRNA (in the vicinity of Cen): 5'-CTGTGGACGTATTAACTTAC-3' (SEQ ID NO:16) + (TGG)-PAM

"Cen" and [Tel] in the sequences described above represent "centromere" and "telomere", respectively.

By the genome editing described above, a human artificial chromosome vector retaining a human centromere and telomere and comprising long-arm moiety and short-arm moiety in which endogenous genes are substantially eliminated may be formed in a human cell. Specific methods and procedures are described in Examples later.

In the case of human chromosomes other than human chromosomes 21 and 13, a human artificial chromosome vector may also be formed by eliminating endogenous genes from a long-arm and a short-arm by a method such as, for example, genome editing similarly in the case of human chromosomes 21 and 13 as described in Examples later. The genome sequence information of human chromosomes may be obtained from databases such as U.S. NCBI.

The human cell comprising the human artificial chromosome vector of interest is, for example, a human cell comprising a pair of homologous human chromosomes and one human artificial chromosome vector originated from a human chromosome homogenous thereto, wherein the human artificial chromosome vector comprises long-arm moiety and short-arm moiety substantially comprising no endogenous gene, a human centromere, and a telomere.

Alternatively, the human cell comprising the human artificial chromosome vector of interest is, for example, a human cell comprising one human chromosome and one or two human artificial chromosome vectors originated from a human chromosome homogenous thereto, wherein the human artificial chromosome vector comprises long-arm moiety and short-arm moiety substantially comprising no endogenous gene, a human centromere, and a telomere, and the cell comprises two copies of a gene for haplo-insufficiency.

A sequence for site-directed DNA insertion and/or an exogenous gene or DNA may be further inserted into the long-arm and short-arm moieties of the artificial chromosome vector described above by using, for example, a genome editing or site-directed recombinant technique.

The human cell comprising the human artificial chromosome vector of interest is collected from a cell population comprising the human cell comprising the human artificial chromosome vector comprising long-arm and short-arm moieties substantially comprising no endogenous gene, a human centromere, and a telomere, produced by the method described above.

In order to isolate a human cell clone comprising the artificial chromosome vector of interest, the above-described cell population is cultured, and a colony is separated. As conditions for culturing human cell, an animal cell, for example, EMEM, DMEM, IMDM, RPMI164, or Ham's F12 medium, or the like may be used as a medium. A medium for primate ES cells is preferably used for culturing differentiated pluripotent stem cells such as iPS cells. The medium may comprise amino acids, vitamins, inorganic salts, trace elements (Cu, Fe, Zn, and the like), nucleosides and bases, substances for energy metabolism (glucose, galactose, Coenzyme A, and the like), antibiotics, cytokines (growth factors, hormones, and the like), supplements, lipids, and optionally serums (fetal bovine serum and the like), which are selected as appropriate. In the case of a serum-free culture medium, a medium comprising such a medium component as described above may be used except that serum is replaced with alternative serum (human serum albumin or the like). As the pH of the medium, pH 7.4 may be usually used for culturing a human cell. Culture temperature is usually 37°C. Culture days are, for example, 5 days or more, and appropriate days are selected. A solid culture, for example, agar or agarose medium is usually used for culture for cloning. The culture may be performed in the presence of 5 to 7% CO₂ in the atmosphere using a container such as a Petri dish, a multi-well plate, or a flask. The culture is described in detail in, for example, R. I. Freshney, Culture of Animal Cells, A Manual of Basic Technique and Specialized, applications, Six Ed., 2010, A John Wiley & Sons, Inc.

A colony comprising the above-described human artificial chromosome vector of interest may be separated by analysis in which, for example, nucleic acids (DNAs) are separated in each of colonies appearing on the medium, and karyotype analysis in which genes are amplified by PCR to identify the elimination of long-arm and short-arm endogenous genes is performed to confirm chromosome destruction as described in Examples later. In addition, a cell clone of interest may be grown and isolated by further solid-culturing and purifying the colony, and then culturing the colony, for example, in a liquid medium comprising the culture components described above.

In the karyotype analysis, each chromosome in a chromosome sample in G-phase in a cell division cycle (G-band chromosome analysis) and/or a chromosome sample in M-phase in a cell cycle (m-band chromosome analysis; using, for example, a mitotic inhibitor such as colchicine or colcemid) may be identified based on a band pattern, a shape, or the like characteristic to chromosomes by using, for example, a Giemsa staining method or a FISH method, and the aberration or the like of the structure, shape, or the like of a chromosome, such as ploidy, aneuploidy, or the presence or absence of translocation may be analyzed.

### 2. Human Cell Comprising Human Artificial Chromosome Vector

In a second aspect according to the present invention, a human cell comprising a human artificial chromosome vector described below is provided.
(1) A human cell comprising a pair of homologous human chromosomes and one human artificial chromosome vector originated from a human chromosome homogenous thereto, wherein the human artificial chromosome vector comprises long-arm and short-arm moieties containing substantially no endogenous gene, a human centromere, and a telomere.
(2) A human cell comprising one human chromosome and one or two human artificial chromosome vectors originated from a human chromosome homogenous thereto, wherein the human artificial chromosome vector comprises long-arm and short-arm moieties substantially containing no endogenous gene, a human centromere, and a telomere, and the cell comprises two copies of a gene for haplo-insufficiency.

The haplo-insufficiency is described in the above-described section 1. When a human chromosome comprises a gene for haplo-insufficiency, it is necessary for maintaining the survival of a cell comprising the chromosome that the cell comprises two copies of a gene for haplo-insufficiency. Therefore, the human cell of the above (2) comprises two copies of a gene for haplo-insufficiency. In such case, the human cell may comprise two copies of a gene for haplo-insufficiency in one human chromosome by, for example, translocation; or a responsible gene for haplo-insufficiency may be found, an exogenous gene vector carrying a gene or DNA for correcting the responsible gene may be produced, and the vector may be introduced into the human cell comprising the human artificial chromosome vector of interest described above to allow two copies of a gene for haplo-insufficiency to be contained in the cell. Such vectors may preferably be vectors that can be used in humans, such as virus vectors (for example, adeno-associated virus, Sendai virus, and the like) and plasmid vectors.

The human cells described above may be produced by, for example, methods described in the above-described section 1 and Examples later.

The human artificial chromosome vector described above may comprise a sequence for site-directed DNA insertion, or may comprise any exogenous gene or DNA. The vector may optionally further comprise at least one of reporter genes and selectable marker genes. Examples of the genes are described in the above-described section 1. Such a human cell may comprise a human artificial chromosome vector comprising an exogenous gene or DNA useful in, for example, gene therapy or the like, and originated from a human.

The gene therapy includes, without limitation, therapeutic methods in which the degradation and insufficiency of protein functions are resolved by genomic gene mutation, therapeutic methods for acquired diseases such as cancers and neurodegenerative diseases, and the like.

The human cell of the present invention is, for example, such a cell as described in the above-described section 1, a somatic cell or a stem cell. A preferred stem cell originated from a human is an iPS cell or a mesenchymal stem cell.

Stem cells originated from humans, such as iPS cells and mesenchymal stem cells comprising the human artificial chromosome vector are useful for gene therapy/regenerative medicine because various progenitor cells and differentiated cells may be produced by differentiation induction from the cells. The human cells of the present invention also include such progenitor cells, differentiated cells, and other cells.

### Examples

The present invention is further specifically described with reference to the following examples. However, the technical scope of the present invention is not limited to these examples.

### [Example 1] Design of Guide RNA for Eliminating All Gene Regions on Long-Arm of Human Chromosome 21

In the present example, guide RNA (gRNA) sequences that may form Clustered Regularly Interspaced Short Palindromic Repeats (CRISPR)/Cas9 that can sequence-specifically cleave a DNA double chain were designed between POTED gene start point (chr21: position 13,609,777) and long-arm start point (chr21: position 12,915,808) downstream of the centromere, and between PRMT2 gene end point (chr21: positions 46,665,685) and telomere sequence start point (chr21: position 46,699,983) in order to eliminate all the gene regions between POTED (chr21: position 13,609,777-13,645,823), the closest translated gene to the centromere, and PRMT2 (chr21: position 46,635,156-46,665,685), the closest translated gene to the telomere, in the long-arm (chr21: position 12,915,808-46,699,983) of human chromosome 21. The version of the genome database is according to GRCh38/hg38 which is the currently available latest version. As a gRNA for cleaving the vicinity of the centromere, gRNA-21q-01 was designed. As a gRNA for cleaving the vicinity of the telomere, gRNA-21q-TeloT9 was designed. The target sequence of each gRNA is as follows.
gRNA-21q-01: 5'-GACAGGTAAGGATTACTAGGAGG-3' (SEQ ID NO:5)
gRNA-21q-TeloT9: 5'-TAAAGATGTGCTCCTTCCCGAGG-3' (SEQ ID NO:6)
gRNAs that may target these sequences were produced by crRNAXT artificial synthesis service in Alt-R (registered trademark) CRISPR-Cas9 crRNA provided by Integrated DNA Technologies, Inc., U.S.

### [Example 2] Evaluation of Cleavage Activity of Telomere Guide RNA for Eliminating All Gene Regions on Long-Arm of Human Chromosome 21

In order to investigate that crRNA comprising a target sequence specified by gRNA-21q-TeloT9 has cleavage activity specifically to the target sequence, a ribonucleoprotein complex ("RNP") composed of a CRISPR-Cas9 protein was introduced into a human iPS cell 201B7 (HPS0063 strain from Cell Engineering Division in RIKEN BioResource Center) by the technique described as follows, and the cell was subjected to T7 endonuclease analysis. First, in gRNA-21q-TeloT9, a crRNAXT product was dissolved in ultrapure water at 200 µM (final concentration). Then, 5 µL of 200 µM gRNA-21q-TeloT9 and 5 µL of 200 µM tracRNA (Integrated DNA Technologies) were mixed, and heated at 95°C for 3 minutes to form a CRISPR-Cas9 protein complex ("RNP"). After the heating, the temperature was decreased to room temperature. In addition, 10.5 µL of PBS, 6 µL of guide RNA, 8.5 µL of Cas9 enzyme were added, gently pipetted, and mixed. The mixture was left at room temperature for 15 minutes to form RNP. As for cells, 201B7 was cultured in a 6-well plate (ThermoFisher) using StemFit AK02N (REPROCELL Inc.) and iMatrix-511 Silk (MATRIXOME, Inc.). In advance, 10 µM Y-27632 (FujifilmWako) was added into a culture medium. The cells were cultured at 37°C for 1 hour. Then, 2 × 10⁶ of 201B7 cells were collected as cells to be introduced, suspended in 10 mL of Opti-MEM (ThemoFisher), and centrifuged at 700 rpm for 10 min, and the cells were washed. Electroporation was used to introduce RNP into 201B7, and Super Electroporator NEPA21 as a product from Nepa Gene Co., Ltd. was used as an instrument. In electroporation conditions, a voltage of 150 V, a pulse length of 5.0 msec, a pulse interval of 50.0 msec, the number of pulses of 2, a decay rate of 10%, and a polarity of ± were set for a poring pulse; and a voltage of 20.0 V, a pulse length of 50.0 msec, a pulse interval of 50.0 msec, the number of pulses of 5, a decay rate of 40%, and a polarity of ± were set for set parameters. As for the cells described above, an Opti-MEM supernatant was removed and the precipitates of the cells was retained, an RNP solution was added and mixed, and the mixture was transferred to a NEPA cuvette electrode set. After electroporation, 201B7 was seeded in 2 mL of StemFit AK02N medium supplemented with 10 µM Y-27632 and 4.8 µL of iMatrix-511 silk in one well of a 6-well plate. The medium was replaced with a usual StemFit AK02N medium 1 day after the electroporation. Genomic DNA originated from the electroporated 201B7 cells was extracted 2 days after the electroporation, and amplification by a PCR method was performed using the genome as a template and using Re-21q tel F3 and Re-21q tel R3. The PCR amplification was performed by using about 100 ng of genomic DNA. KOD One^{(R)} PCR Master Mix manufactured by TOYOBO CO., LTD. was used as DNA polymerase, and reaction conditions were denaturing at 98°C for 10 seconds, annealing at 60°C for 5 seconds, and elongation at 68°C for 5 seconds, and for 35 cycles. The amplified product was electrophoresed with a 2% agarose gel, then stained with ethidium bromide to detect. As a result, the amplified product having an expected length (507 bp) was detected. The amplified product was subjected to T7 endonuclease analysis using T7 Endonuclease I reaction Mix (NIPPON GENE CO., LTD.). Primers were produced using Primer3Plus based on the base sequences obtained from the database of NCBI. The names and sequences of the primers are described as follows.
Re-21q tel F3: 5'-AACTGAGCCCCTTTCTTCGG-3' (SEQ ID NO:7)
Re-21q tel R3: 5'-AGGATTCTCTGCTCCCCCAT-3' (SEQ ID NO:8)

The T7 endonuclease analysis was performed by the following technique.

The denaturing and annealing operations of the amplified product were performed in a thermal cycler in which the PCR solution comprising the amplified product was loaded. Reaction is performed using 8 µL of amplified product to which 1 µL of 1M NaCl was added, and the reaction conditions of denaturing at 95°C for 5 minutes, and the annealing at 2°C/sec at 95°C to 85°C, and at 0.1°C/sec at 85°C to 25°C. The resultant was mixed with 9 µL of matrix DNA fragment and 1 µL of T7 Endonuclease I reaction Mix (T7E), and the mixture was reacted at 37°C for 1 hour. The reaction product was electrophoresed with a 2% agarose gel, then stained with ethidium bromide to detect. As a result, the reaction product having an expected length (before cleavage: about 507 bp, after cleavage: about 364 bp, about 143 bp) was detected (Figure 1). Lane 1 (RNP+, T7E+) in Figure 1 shows the result of the PCR product of DNA originated from an RNP-introduced cell with T7E being added. Lane 2 (RNP+, T7E-) shows the result of the PCR product of DNA originated from an RNP-introduced cell without T7E being added. Lane 3 (RNP-, T7E+) shows the result of the PCR product of DNA originated from an RNP-unintroduced cell with T7E being added. Lane 4 (RNP-, T7E-) shows the result of the PCR product of DNA originated from an RNP-unintroduced cell with T7E being added. Lane 5 (Marker) shows a DNA size index marker, Gene ladder wide 2 (NIPPON GENE CO., LTD.). Based on the results, gRNA-21q-TeloT9 was used because cleavage efficiency thereof was about 12.19%. gRNA-21q-01 was used without evaluating cleavage activity efficiency, on the other hand.

### [Example 3] Introduction of gRNA into Human iPS Cell Strain for Eliminating Long-Arm of Human Chromosome 21

crRNAXT, gRNA-21q-TeloT9 for cleaving the vicinity of the telomere of the long-arm of human chromosome 21 of which the design and cleavage activity evaluation were performed in Examples 1 and 2, and gRNA-21q-01 for cleaving the vicinity of the centromere of the long-arm of human chromosome 21 were introduced into a human iPS cell strain 201B7 by electroporation. As a method, first, 2.5 µL of gRNA-21q-TeloT9, 2.5 µL of 200 µM gRNA-21q-01, and 5 µL of 200 µM tracRNA were mixed in the same microtube, and heated at 95°C for 3 minutes. After the heating, the temperature was decreased to room temperature. Then, the same technique as that of the CRISPR-Cas9-RNP electroporation described in Example 2 was performed. Subsequently, the human iPS cells into which RNP was introduced were cultured for 2 days (48 hours) and 1 week, genomic DNAs were then extracted, and amplified by a PCR method using the genomes as templates, and using a forward primer 21q-01 F (5'-GTGCAACTGTCAGACCAAAACA-3' (SEQ ID NO:9)) specific for the long-arm of human chromosome 21 in the vicinity of the centromere and a reverse primer Re-21q tel R3 (SEQ ID NO:8) specific for the long-arm of human chromosome 21 in the vicinity of the telomere. The amounts of the template DNAs used per reaction in the PCR amplification were set at 100 ng, 10 ng, and 1 ng. Reaction was performed using KOD One^{(R)} PCR Master Mix manufactured by TOYOBO CO., LTD. as DNA polymerase, and the conditions of denaturing at 98°C for 10 seconds, annealing at 65°C for 5 seconds, elongation at 68°C for 5 seconds, and for 35 cycles. Regarding the forward primer specific for the long-arm of human chromosome 21 in the vicinity of the centromere, the name of the primer and the sequence of SEQ ID NO:9 are described as follows.
21q-01 F: 5'-GTGCAACTGTCAGACCAAAACA-3' (SEQ ID NO:9)

The amplified products were electrophoresed with a 2% agarose gel, then stained with ethidium bromide to detect. For the genomic DNAs after 1 week, the expected amplified products (about 245 bp) were confirmed at 100 ng and 10 ng (Figure 2). A DNA ladder marker, Gene ladder wide 2, was set as lane 1 (Marker). Lane 2 (201B7 wt) shows the PCR result using DNA originated from untreated wild type 201B7. Lanes 3 to 5 (after 48 H; 100 ng, 10 ng, and 1 ng) show the results of analysis using genomic DNAs collected 48 hours after the introduction of a combination of gRNAs for eliminating the long-arm of chromosome 21 into 201B7. The obtained PCR results showed that specific sequences generated when the long-arm of chromosome 21 was eliminated as intended (amplified product specific for long-arm of chromosome 21 being eliminated) appeared 48 hours after the introduction of RNP when template DNAs equivalent to 100 ng, 10 ng, and 1 ng of DNAs were used. The efficiency of eliminating the long-arm of chromosome 21 can be calculated based on the amount of template DNA used in which the amplified product was detected. The amplified product specific for chromosome 21 with the long-arm being eliminated was detected when 1 ng of template DNA (template DNA equivalent to about 150 cells) was used. Therefore, it is considered that the long-arm of chromosome 21 was eliminated in at least one cell of the 150 cells. This suggested that, in the case of 1 ng which was the minimum amount of template DNA used, the long-arm of chromosome 21 was eliminated at a frequency of one or more cells in about 150 cells equivalent to 1 ng of DNA. Lanes 6 to 8 (after 1 week; 100 ng, 10 ng, and 1 ng) show the results of analysis using genomic DNAs collected 1 week after the introduction of a combination of gRNAs for eliminating the long-arm of chromosome 21 into 201B7. It was suggested that the long-arm of chromosome 21 was eliminated in one or more cells (0.67%) of about 150 cells equivalent to 100 ng, 10 ng, or 1 ng of DNA 48 hours after the introduction of RNP. When the culture was further continued for 1 week and detection specific for chromosome 21 with the long-arm being eliminated was performed using DNAs following the growth of the cells as template DNAs, expected specific recombinant bands were detected in the case of using template DNAs in amounts of 100 ng and 10 ng. This suggested that the long-arm of chromosome 21 was eliminated in one or more cells (0.067%) out of about 1500 cells.

### [Example 4] Obtainment of Clone in which Long-Arm of Human Chromosome 21 is Eliminated, and Determination of Elimination of Long-Arm Region by PCR Method

In order to isolate and establish a cell strain, in which a long-arm region was eliminated, from an RNP-introduced 201B cell population for eliminating a long-arm region in Example 2, an RNP-introduced 201B7 cell population for eliminating a long-arm region was seeded to 96 wells so that 10 cells were put per well in a 96-well plate, and about 960 cells were sorted. After culturing for 7 to 9 days, cells were collected from each well when the growth of the cells therein was observed. For a method of the collection, the cells were washed with 100 µL of PBS supplemented with 1 mM EDTA, and left at 37°C for 5 minutes after100 µL of PBS supplemented with 1 mM EDTA being added thereto, and a supernatant was then removed. The cells were further washed with 100 µL of PBS, and then suspended in 25 µL of PBS supplemented with Y-27632. In a 96-well plate, 15 µL of the cell solution was subcultured. Then, 10 µL of the cell solution was mixed with 10 µL of Proteinase K solution, and the mixture was kept warm at 55°C overnight and then heated at 95°C for 3 minutes to extract genomic DNA. as the Proteinase K solution, a mixture of 471 µL of sterilized water, 25 µL of ExTaq Buffer (TAKARA), and 4 µL of 10 mg/mL Proteinase K (Fujifilm Wako) was used. Amplification by a PCR method was performed by using the obtained genomic DNA as a template and using Re-21q tel R3 (SEQ ID NO:8) and 21q-01 F (SEQ ID NO:9) as PCR primers for specifically detecting the elimination of the long-arm of chromosome 21. As a result, when DNA originated from a cell at position 6F in a 96-well plate was used as a template, an expected amplified product (245 bp) was confirmed as shown in lane 10 (Figure 3). As a positive control (PC), DNA originated from a cell population 48 hours after the introduction of RNP was used as a template. Lane 1 (Marker) shows a DNA size index marker, Gene ladder wide 2 (NIPPON GENE CO., LTD.). An amplified product specific for chromosome with the long-arm of being eliminated was not obtained when DNAs originated from positions other than 6F were used, as shown in lanes 2 to 9, and 11 to 13 in turn from the right of lane 1.

In contrast, for evaluation of the presence of the inversion of the long-arm of chromosome 21, amplification by a PCR method was performed using 21q-01 R (SEQ ID NO: 10) which was a reverse primer specific for the long-arm of human chromosome 21 in the vicinity of the centromere and Re-21q tel F3 (SEQ ID NO:7) which was a forward primer specific for the long-arm of human chromosome 21 in the vicinity of the telomere, for specifically detecting the inversion of the long-arm of chromosome 21, which was expected to occur upon elimination of the long-arm of chromosome 21. As a result, when DNA originated from the position 6F was used, an amplified product specific for the occurrence of the inversion of the long-arm of chromosome 21 was not detected (Figure 4). Regarding the reverse primer specific for the long-arm of human chromosome 21 in the vicinity of the centromere, the name of the primer and the sequence of SEQ ID NO: 10 are described as follows.
21q-01 R: 5'-TGCCTGACTGAAAAGATGAGT-3' (SEQ ID NO: 10)

### [Example 5] Obtainment of Clone in which Long-Arm of Human Chromosome 21 is Eliminated, and Determination of Elimination of Long-Arm Region by PCR Method

The results of Example 4 suggested that, in the cell population at the position 6F (cell population 6F), a population in which the full length of the long-arm of chromosome 21 was eliminated was present. Based on the results, cells in which the long-arm of chromosome 21 was eliminated were cloned. Since the cell population originated from the position 6F was a population formed from around 10 cells, 150 cells per plate were seeded in a 96-well plate in order to single-clone the cells. A generated colony was picked up after 10 days. A method of the pick-up was the same as the -technique using the Proteinase K solution described above. Amplification by a PCR method was performed using genomic DNA obtained from the 96-well plate as a template and using primers Re-21q tel R3 (SEQ ID NO:8) and 21q-01 F (SEQ ID NO:9) which can detect the elimination of the long-arm of human chromosome 21. As a result, expected amplified products (245 bp) were confirmed for 12 subclones among 49 subclones (Figure 5). Based on the above, the efficiency of the elimination of chromosome 21 in the population in which RNP-introduced 201B7 was cultured for 1 week was concluded to be 0.03% since, when the efficiency of the elimination of the long-arm of human chromosome 21 in the case of using 201B7 was considered sequentially from the beginning, 10 cells were seeded in 96 wells, a PCR amplified product specific for human chromosome 21 with the long-arm being eliminated was confirmed in 1 well out of 96 wells, 49 subclones were further screened by subcloning, and PCR amplified products specific for chromosome 21 with the long-arm being eliminated were finally obtained in 12 subclones (lanes #3 to #38 in the figure).

### [Example 6] Evaluation of Improvement in Efficiency of Elimination of Long-Arm of Human Chromosome 21 in Human 21 Trisomic iPS Cell Strain

The long-arm of chromosome 21 was eliminated using the iPS cell strain 2767-4 originated from a human patient with Down's syndrome, which is a trisomic syndrome having three chromosomes 21, while a human cell is normally a disomy containing two chromosomes 21. As in Example 2, RNP for eliminating the long-arm of chromosome 21 was introduced by an electroporation method, DNA was collected 48 hours and 1 week after the introduction of the RNP, and the presence or absence of an amplified product specific for chromosome 21 with the long-arm being eliminated was investigated by a PCR method. As a result, when the trisomic 21 cell strain 2767-4 was used, amplified products expected (245 bp) were confirmed in the case of using template DNAs equivalent to 100 ng, 10 ng, and 1 ng for DNAs originated from RNP-introduced cells after 48 hours (After 48H) and 1 week (After 1 week) (Figure 6). In contrast, when a disomic strain 201B7 was used, the expected amplified products (245 bp) were confirmed in the case of using the template DNAs equivalent to 100 ng, 10 ng, and 1 ng for DNAs originated from RNP-introduced cells obtained 48 hours after the introduction of RNP (After 48H) (Figure 2). However, in the case using a template DNA equivalent to 1 ng, an expected amplified product was not observed for the DNAs originated from the RNP-introduced cells (Figure 2). As a result, the frequency of generation of a cell in which the long-arm of chromosome 21 was eliminated was about one per 150 cells when the trisomic cell strain was used. The frequency of generation of a cell in which the long-arm of chromosome 21 was eliminated was about one per 1500 cells when the human iPS cell strain 201B7 was used. This suggested that the efficiency of elimination of the long-arm of chromosome 21 in the case of using the trisomic cell strain was 10 times compared to the case of using the human iPS cell strain 201B7.

### [Example 7] Investigation of Elimination of Long-Arm of Chromosome 21 by G-Band Chromosome Analysis

In order to identify elimination of the long-arm of chromosome 21 in the subclone of the cell population 6F obtained in Example 4 by a cytogenetic technique, G-band chromosome analysis was performed by a GTG method. Expansion culture of two subclones (#10 and #29) out of 12 subclones was performed in a 6-cm dish for 11 to 13 days. Appropriate amounts of Metaphase Arresting Solution (Genial Genetic Solutions limited) and Chromosome Resolution Additive (Genial Genetic Solutions limited) were added into culture media containing cells in a logarithmic growth phase so that the final concentrations thereof were 0.25 µg/mL and 0.1 µL/mL, respectively, and the cells were cultured at 37°C for 90 minutes. The cells were collected, then suspended in 5 mL of 0.075M KCl, and treated with a hypotonic solution at room temperature for 15 minutes. After centrifugation at 1500 rpm for 5 minutes, a supernatant was removed, and the resultant was suspended in 5 mL of Carnoy's solution in which acetic acid and methanol were mixed to be 1: 3, to produce a Carnoy fixed floating sample. The Carnoy fixed floating sample was spread on slide glass, trypsinized with ice-cooled 0.05% trypsin solution for 20 to 50 seconds, and then Giemsa-stained with 2% Giemsa's stain solution. Metaphase cells were searched with an electrically driven upright microscope AxioImagerZ2 (CarlZeiss microscopy) comprising a CoolCube1m CCD camera (MetaSystems) and Metafer Slide Scanning System software (MetaSystems), and G-band chromosome analysis of randomly selected 20 cells was performed with Ikaros Karyotyping System software (MetaSystems). Compared to wild type 201B7 with normal karyotype, ?del (21) (q11.2q22.3), chromosome 21 with the partial deletion of the long-arm considered to be caused by the intended elimination of the long-arm, was detected in all the analyzed 20 cells for both #10 and #29. Derived chromosome 21 with an additional segment in the other end of the long-arm of chromosome 21, der (21) was detected, and the fringe pattern of the G-band observed in the additional segment suggested that inversion insertion (or translocation) of a chromosome region 21q1 1.2 to q22.3, eliminated in ?del (21) (q11.2q22.3), occurred in der (21). Other aberration related to the number of chromosomes and structural aberration were not detected (Figures 7-1 to 7-3).

### [Example 8] Investigation of Elimination of Long-Arm of Chromosome 21 by mBand Chromosome Analysis

Regarding the chromosome structural aberration detected in Example 5, ?del (21) (q11.2q22.3) and der (21), mBand chromosome analysis of two subclones (#10 and #29) was performed to identify the details of structural aberration such as a breakpoint. The Carnoy fixed floating sample produced in Example 5 was spread on slide glass, and subjected to staining treatment using Human mBAND Probes XCyte 21 (MetaSystems) according to the protocol attached to the product. As in Example 5, metaphase cells were searched, and mBand chromosome analysis of randomly selected 3 cells was performed with Isis FISH Imaging System software (MetaSystems). In all the analyzed 3 cells for each of #10 and #29, chromosomes with the DAPI images as those of ?del (21) (q11.2q22.3) and der (21) detected in Example 5 were detected, and comparison with mBand signals observed in the normal chromosome 21 of wild type 201B7, which is simultaneously treated for staining, confirmed that a chromosome region 21q11.2 to q22.3 was deleted in ?del (21) (q11.2q22.3), and a chromosome region 21q11.2 to q22.3 was inversely added to the end of the long-arm in der (21) (Figures 8-1 to 8-3). In the figure, the upper section shows the results of the mBand chromosome analysis of two chromosomes 21, and the middle and lower sections show the detailed results of each chromosome, respectively. Based on the results, it was concluded that the chromosome structural aberration in der (21) was the structural aberration ?ins (21; 21) (q22.3; q22.3q11.2), which is caused by the chromosome region 21q11.2 to q22.3, which was eliminated in ?del (21) (q1 1.2q22.3), being inserted inversely or translocated into the end of the long-arm of chromosome 21, and that ?del (21) (q11.2q22.3), which was expected to be caused by eliminating the long-arm of chromosome 21, was obtained.

### [Example 9] Obtainment of Clone, in Which Long-Arm of Chromosome Human 21 is Eliminated, with Improved Efficiency of Elimination of Long-Arm of Human Chromosome 21 in Human 21 Trisomic iPS Cell Strain (2767-4), And Determination of Elimination of Long-Arm Region by PCR Method

As in Example 3, in order to isolate and establish a cell strain, in which a long-arm region was eliminated, from a 2767-4 cell population into which RNP was introduced for eliminating a long-arm region, a 2767-4 cell population into which RNP was introduced was seeded to 96 wells so that 10 cells were put per well in a 96-well plate, and about 960 cells were sorted. After culturing for 7 to 9 days, cells were collected from each well when the growth of the cells therein was observed and genomic DNA was extracted while the culture was continued for a part thereof. Amplification by a PCR method was performed by using the obtained genomic DNA as a template and using Re-21q tel R3 (SEQ ID NO:8) and 21q-01 F (SEQ ID NO:9) as PCR primers for specifically detecting the elimination of the long-arm of chromosome 21. As a result, expected amplified products were obtained in 5 wells (well Nos. 59, 64, 66, 71, and 78) out of 96 wells (Figure 9). In the figure, lanes 2 to 17 in each gel show the results of clones at positions with different 96-well numbers. Likewise, when the inversion of the long-arm of chromosome 21 was determined, amplified products specific therefor were not detected in all the 5 wells (well Nos. 59, 64, 66, 71, and 78) (Figure 10). Based on the results, the efficiency of elimination of the long-arm of human chromosome 21 in a trisomic cell strain 2767-4 was a probability of 5 wells out of 96 wells. Based on Example 3, the efficiency of elimination of the long-arm of human chromosome 21 was 1 well out of 96 wells when a disomic strain 201B7 was used. Based on the above, the efficiency of elimination of the long-arm of chromosome 21 was considered to be increased by 5 times compared to the case of using the trisomic cell strain.

Based on the results, in order to confirm the presence of a population in which the full length of the long-arm of chromosome 21 was eliminated in the cell populations of well Nos. 59, 64, 66, 71, and 78, a cell in which the long-arm of chromosome 21 was eliminated using a trisomic cell strain was cloned. Since the cell population originated from well No. 59 (cell population 59) was a population formed from around 10 cells, 150 cells per plate were seeded in a 96-well plate in order to single-clone the cells. A generated colony was picked up after 10 days. A method of the pick-up was the same as the technique using the Proteinase K solution described above. Amplification by a PCR method was performed using genomic DNA obtained from the 96-well plate as a template and using primers Re-21q tel R3 (SEQ ID NO:8) and 21q-01 F (SEQ ID NO:9) which can detect the elimination of the long-arm of human chromosome 21. As a result, expected amplified products (245 bp) were confirmed in 4 subclones (#8, 17, 24, and 26) out of 26 subclones (Figure 11). In the figure, "#6F #29" shows the result for the subclone #29 of the cell population 6F of the disomic cell strain 201B7 cell being used. Based on the above, the efficiency of the elimination of chromosome 21 in the population in which RNP-introduced 2767-4 was cultured for 1 week was concluded to be 0.08% since, when the efficiency of the elimination of the long-arm of human chromosome 21 in the case of using the 2767-4 cell was considered sequentially from the beginning, 10 cells were seeded in 96 wells, a PCR amplified product specific for human chromosome 21 with the long-arm being eliminated was confirmed in 5 wells out of 96 wells, 26 subclones were further screened by subcloning, and PCR amplified products specific for chromosome 21 with the long-arm being eliminated were finally obtained in 4 subclones.

### [Example 10] Investigation of Elimination of Long-Arm of Trisomic Chromosome 21 by Chromosome Analysis by DAPI Staining

In order to identify the elimination of the long-arm of chromosome 21 by a cytogenetic technique, DAPI chromosome analysis of the subclones of the cell population 59 obtained in Example 9 was performed. Each subclone was expansion-cultured in a 6-cm dish, and a Carnoy fixed floating sample was then produced to spread on slide glass. A technique used from the expansion culture to a Carnoy fixation method was the same as the technique performed for the G-band chromosome analysis described above. In a DAPI (4',6-diamidino-2-phenylindole) staining technique, SlowFadeTM Gold antifade reagent with DAPI (Invitrogen) supplemented with 1 µg/mL of DAPI (DOJINDO LABORATORIES) was applied onto the chromosome sample, and the sample was mounted with cover glass. Q-band chromosome analysis of randomly selected 20 cells was performed by microscopic examination. As in Example 5, chromosome aberration expected to occur due to the elimination of the long-arm of chromosome 21 was detected and evaluated by this technique. In the subclones #59-26 (subclone 26 of cell population 59), 2 normal chromosomes 21 and chromosome 21 with partial deletion of a long-arm considered to be caused by the intended elimination of the long-arm were observed in comparison with the trisomic cell strain 2767-4 from which the subclones were originated (Figure 12).

### [Example 11] Introduction of gRNA into Human iPS Cell Strain for Eliminating Short-Arm of Human Chromosome 13

By electroporation, crRNAXT, gRNA-13p-TeloT3 (5'-AGAAGCGAATCCTGCTTGCA-3' (SEQ ID NO:4) + (GGG)) for cleaving the vicinity of the telomere of the short-arm of human chromosome 13 and gRNA-13p-T142 (5'-AGAACTGAAGCATCACTCAC-3' (SEQ ID NO:3) + (TGG)) for cleaving the vicinity of the centromere of the short-arm of human chromosome 13 were introduced into a human iPS cell strain 201B7. As a method therefor, first, 2.5 µL of gRNA-13p-TeloT3, 2.5 µL of 200 µM gRNA-13p-T142, and 5 µL of 200 µM tracRNA were mixed in the same microtube, and heated at 95°C for 3 minutes. After the heating, the temperature was decreased to room temperature. Then, the same technique as that of the CRISPR-Cas9-RNP electroporation described in Example 2 was performed. The target sequence of each gRNA is as follows.
gRNA-13p-T142: 5'-AGAACTGAAGCATCACTCAC-3' (SEQ ID NO:3) + (TGG)
gRNA-13p-TeloT3: 5'-AGAAGCGAATCCTGCTTGCA-3' (SEQ ID NO:4) + (GGG)

Subsequently, the human iPS cells into which RNP was introduced were cultured for 2 days (48 hours) and 1 week, and then genomic DNAs were extracted, and amplified by a PCR method using the genomes as templates, and using a forward primer 13p-T142R (5'-TGACAGGCATCCATGGGAAC-3' (SEQ ID NO:11)) specific for the short-arm of human chromosome 13 in the vicinity of the centromere and a reverse primer Re-Chr13p tel F2 (5'-CTCAGACCCGGAGCTTTCTC-3' (SEQ ID NO:12)) specific for the short-arm of human chromosome 13 in the vicinity of the telomere. The amounts of the template DNAs used per reaction in the PCR amplification were set at 100 ng, 10 ng, and 1 ng. Reaction was performed using KOD One^{(R)} PCR Master Mix manufactured by TOYOBO CO., LTD. as DNA polymerase, and the conditions of denaturing at 98°C for 10 seconds, annealing at 65°C for 5 seconds, elongation at 68°C for 5 seconds, and for 35 cycles. The amplified products were electrophoresed with a 2% agarose gel, then stained with ethidium bromide to detect. The names and sequences of the primers are described as follows.
13p-T142R: 5'-TGACAGGCATCCATGGGAAC-3' (SEQ ID NO:11)
Re-Chr13p tel F2: 5'-CTCAGACCCGGAGCTTTCTC-3' (SEQ ID NO:12)

For the genomic DNAs after 1 week, the expected amplified products (about 245 bp) were confirmed at 100 ng and 10 ng (Figure 13). A DNA ladder marker, Gene ladder wide 2, was set as lane 1 from the left. Lane 2 shows the PCR result using DNA originated from wild type 201B7. Lane 3 to lane 5 (after 48 H; 100 ng, 10 ng, and 1 ng) show the results of analysis using genomic DNAs collected 48 hours after the introduction of a combination of gRNAs for eliminating the short-arm of chromosome 13 into 201B7. The obtained PCR results showed that specific sequences generated when the short-arm of chromosome 13 was eliminated as intended appeared 48 hours after the introduction of RNP when template DNAs equivalent to 100 ng, 10 ng, and 1 ng of DNAs were used. Lane 3 to lane 8 show the results of analysis using genomic DNAs collected 48 hours (After 48 H) and 1 week (After 1 week) after the introduction of a combination of gRNAs for eliminating the short-arm of chromosome 13 into 201B7. It was suggested that the short-arm of chromosome 13 was eliminated in one or more cells (0.0067%) out of about 15000 cells, which is equivalent to DNA for 100 ng of DNA, 48 hours after the introduction of RNP. When the culture was further continued for 1 week and detection specific for chromosome 13 with the short-arm being eliminated was performed using DNAs after the growth of the cells as template DNAs, specific recombinant bands as expected were detected when template DNAs are used in amounts of 100 ng and 10 ng. This suggested that the short-arm of chromosome 13 was eliminated in one or more cells (0.067%) out of about 1500 cells (equivalent to DNA for 10 ng of DNA).

### [Example 12] Obtainment of Clone in which Short-Arm of Human Chromosome 13 is Eliminated, and Determination of Elimination of Short-Arm Region by PCR Method

In order to isolate and establish a cell strain, in which a short-arm region was eliminated, from an RNP-introduced 201B cell population for eliminating a short-arm region in Example 11, an RNP-introduced 201B7 cell population for eliminating a short-arm region was seeded to 96 wells so that 10 cells were put per well in a 96-well plate, and about 960 cells were sorted. After culturing for 7 to 9 days, and cells were collected from each well when the growth of the cells therein was observed. For a method of the collection, the cells were washed with 100 µL of PBS supplemented with 1 mM EDTA, and left to stand at 37°C for 5 minutes after 100 µL of PBS supplemented with 1 mM EDTA being added thereto, and a supernatant was then removed. The cells were further washed with 100 µL of PBS, and the cells were then suspended in 25 µL of PBS supplemented with Y-27632. In a 96-well plate, 15 µL of the cell solution was subcultured. Then, 10 µL of the cell solution was mixed with 10 µL of Proteinase K solution, and the mixture was kept warm at 55°C overnight and then heated at 95°C for 3 minutes to extract genomic DNA. As the Proteinase K solution, a mixture of 471 µL of sterilized water, 25 µL of ExTaq Buffer (TAKARA), and 4 µL of 10 mg/mL Proteinase K (Fujifilm Wako) was used. Amplification by a PCR method was performed by using the obtained genomic DNA as a template and using a forward primer 13p-T142R (5'-TGACAGGCATCCATGGGAAC-3' (SEQ ID NO:11)) specific for the short-arm of human chromosome 13 in the vicinity of the centromere and a reverse primer Re-Chr13p tel F2 (5'-CTCAGACCCGGAGCTTTCTC-3' (SEQ ID NO:12)) specific for the short-arm of human chromosome 13 in the vicinity of the telomere.

As a result, expected amplified products (245 bp) were confirmed when DNAs originated from cells at positions 4B, 4C, 5C, 6E, 6F, 6H, 7D, 8G, 10A, 10C, and 11D in a 96-well plate were used as templates,(Figure 14). As a positive control (PC), DNA originated from a cell population 48 hours after the introduction of RNP was used as a template. Lane 1 (Marker) shows a DNA size index marker, Gene ladder wide 2 (NIPPON GENE CO., LTD.).

In contrast, for evaluation of the presence of the inversion of the short-arm of chromosome 21 amplification by a PCR method was performed using 13p-T142F (5'-GGCTCCATCCCATGTGAGAG-3' (SEQ ID NO:13)) which was a reverse primer specific for the short-arm of human chromosome 13 in the vicinity of the centromere and Chr13p tel F2 (5'-AGAACTGAAGCATCACTCACTGG-3' (SEQ ID NO:14)) which was a forward primer specific for the short-arm of human chromosome 13 in the vicinity of the telomere, for specifically detecting the inversion of the short-arm of chromosome 13, which was expected to occur upon elimination of the short-arm of chromosome 13. As a result, when DNAs originated from the positions 4C and 5C were used, an amplified product specific for the occurrence of the inversion of the short-arm of chromosome 13 was not detected. In contrast, when DNA originated from the position 6E was used, an amplified product specific for the occurrence of the inversion of the short-arm of chromosome 13 was detected (Figure 15). The names of the primers and the sequence of SEQ ID NO:9 are described as follows.
13p-T142F: 5'-GGCTCCATCCCATGTGAGAG-3' (SEQ ID NO:13)
Chr13p tel F2: 5'-AGAACTGAAGCATCACTCACTGG-3' (SEQ ID NO:14)

### [Example 13] Obtainment of Clone in which Short-Arm of Human Chromosome 13 is Eliminated, and Determination of Elimination of Short-Arm Region by PCR Method

The results of Example 12 suggested that, in the cell population at the position 5C (cell population 5C), a population in which the full length of the short-arm of chromosome 13 was eliminated was present. Based on the results, cells in which the short-arm of chromosome 13 was eliminated were cloned. Since the cell population originated from the position 5C was a population formed from around 10 cells, 150 cells per plate were seeded in a 96-well plate in order to single-clone the cells. A generated colony was picked up after 10 days. A method of the pick-up was the same as the technique using the Proteinase K solution described above. Amplification by a PCR method was performed using genomic DNA obtained from the 96-well plate as a template and using a forward primer 13p-T142R (5'-TGACAGGCATCCATGGGAAC-3' (SEQ ID NO:11)) specific for the short-arm of human chromosome 13 in the vicinity of the centromere and a reverse primer Re-Chr13p tel F2 (5'-CTCAGACCCGGAGCTTTCTC-3' (SEQ ID NO:12)) specific for the short-arm of human chromosome 13 in the vicinity of the telomere.

As a result, expected amplified products (245 bp) were confirmed in 11 subclones (indicated #4 to #63) out of 96 subclones (Figure 16). In Figure 16, "5C Mass" shows the result for using the cell population 5C that was not single-cloned. Based on the above, the efficiency of the elimination of chromosome 13 in the population in which RNP-introduced 201B7 was cultured for 1 week was concluded to be 0.0011% since, when the efficiency of the elimination of the short-arm of human chromosome 13 in the case of using 201B7 was considered sequentially from the beginning, 10 cells were seeded in 96 wells, a PCR amplified product specific for human chromosome 13 with the short-arm being eliminated was confirmed in 1 well out of 96 wells, 9 subclones were further screened by subcloning, and PCR amplified products specific for chromosome 13 with the short-arm being eliminated were finally obtained in 3 subclones (#2, 4, and 5) (Figure 17). In Figure 17, "5C #4" shows the result for using a cell population in which further single-cloning of the subclone #4 of the cell population 5C was not performed.

### [Example 14] Investigation of Elimination of Short-Arm of Chromosome 13 by G-Band Chromosome Analysis

In order to identify elimination of the short-arm of chromosome 13 in the subclone of the cell population 5C obtained in Example 13 by a cytogenetic technique, G-band chromosome analysis was performed by a GTG method. Expansion culture of 2 subclones (#4-2 and #10-8) out of 12 subclones was performed in a 6-cm dish for 11 to 13 days. Appropriate amounts of Metaphase Arresting Solution (Genial Genetic Solutions limited) and Chromosome Resolution Additive (Genial Genetic Solutions limited) were added into culture media containing cells in a logarithmic growth phase so that the final concentrations thereof were 0.25 µg/mL and 0.1 µL/mL, respectively, and the cells were cultured at 37°C for 90 minutes. The cells were collected, then suspended in 5 mL of 0.075M KCl, and treated with a hypotonic solution at room temperature for 15 minutes. After centrifugation at 1500 rpm for 5 minutes, a supernatant was removed, and the resultant was suspended in 5 mL of Carnoy's solution in which acetic acid and methanol were mixed to be 1: 3, to produce a Carnoy fixed floating sample. The Carnoy fixed floating sample was spread on slide glass, trypsinized with ice-cooled 0.05% trypsin solution for 20 to 50 seconds, and then Giemsa-stained with 2% of Giemsa's stain solution. Metaphase cells were searched with an electrically driven upright microscope AxioImagerZ2 (CarlZeiss microscopy) comprising a CoolCube1m CCD camera (MetaSystems) and Metafer Slide Scanning System software (MetaSystems), and G-band chromosome analysis of randomly selected 20 cells was performed with Ikaros Karyotyping System software (MetaSystems). Compared to wild type 201B7 cells with normal karyotype, del (13) (p11.2), chromosome 13 with the partial deletion of the long-arm considered to be caused by the elimination of the short-arm as intended, was detected in all the analyzed 20 cells in both #4-2 and #10-8 (Figure 18).

### [Example 15] Introduction of gRNA into Human iPS Cell Strain for Elimination of Long-Arm of Human Chromosome 13

As in Example 11, gRNA for specifically cleaving the vicinity of the centromere or the vicinity on the side of telomere of the long-arm of human chromosome 13 can be designed and introduced into a disomic human iPS cell strain or trisomic iPS cell strain to eliminate the full length of a long-arm region.

### [Example 16] Obtainment of Clone in which Long-Arm of Human Chromosome 13 is Eliminated, and Determination of Elimination of Short-Arm Region by PCR Method

As in Example 12, for a cell in which gRNA for eliminating the long-arm of human chromosome 13 was introduced into a disomic human cell strain and a trisomic human cell strain in Example 15 for human chromosome 13, the degree of appearance of a cell strain in which the long-arm of human chromosome 13 was eliminated in a cell population may be examined by a PCR method using a primer that can specifically detect the long-arm of elimination.

### [Example 17] Obtainment of Clone in which Long-Arm of Human Chromosome 13 is Eliminated, and Determination of Elimination of Long-Arm Region by PCR Method

As in Example 13, cell clones obtained from each well may be sorted by a PCR method using a probe for specifically indicating the elimination of a long-arm to obtain a single clone in which the long-arm of human chromosome 13 was eliminated.

### [Example 18] Investigation of Elimination of Long-Arm of Chromosome 13 by G-Band Chromosome Analysis

As described in Example 7, in order to identify the elimination of the long-arm of chromosome 13 in the produced subclone by a cytogenetic technique, a Carnoy fixed floating sample prepared as in Example 5 was spread on slide glass, trypsinized with ice-cooled 0.05% trypsin solution for 20 to 50 seconds, and then Giemsa-stained with 2% of Giemsa's stain solution. Metaphase cells are searched with an electrically driven upright microscope AxioImagerZ2 (CarlZeiss microscopy) comprising a CoolCube1m CCD camera (MetaSystems) and Metafer Slide Scanning System software (MetaSystems), and G-band chromosome analysis of the subclone for randomly selected cells may be performed with Ikaros Karyotyping System software (MetaSystems).

### [Example 19] Investigation of Elimination of Long-Arm of Chromosome 13 by m-Band Chromosome Analysis

As described in Example 8, regarding the chromosome structural aberration upon the elimination of the long-arm of the chromosome 13 of produced subclones, mBand chromosome analysis of the produced subclones is performed to identify the details of structural aberration such as a breakpoint. The Carnoy fixed floating sample, which is produced as in Example 5, is spread on slide glass, and subjected to staining treatment using Human mBAND Probes XCyte 21 (MetaSystems) according to the protocol attached to the product. As in Example 5, metaphase cells are searched, and mBand chromosome analysis of the subclones for randomly selected cells can be performed with Isis FISH Imaging System software (MetaSystems).

### [Example 20] Evaluation of Improvement in Efficiency of Elimination of Short-Arm of Human Chromosome 21 in Human 21 Trisomic iPS Cell Strain

By electroporation, crRNAXT, gRNA-21p-TeloT6 (5'-AGACTGTAGCCTTGACCCAG-3' (SEQ ID NO: 15) + (AGG)) for cleaving the vicinity of the telomere of the short-arm of human chromosome 21 and gRNA-21p-T94 (5'-CTGTGGACGTATTAACTTAC-3' (SEQ ID NO: 16) + (TGG)) for cleaving the vicinity of the centromere of the short-arm of human chromosome 21 were introduced into a human iPS cell strain 201B7 and human 21 trisomic iPS cell strain 2767-4. As a method therefor, first, 2.5 µL of gRNA-21p-TeloT6, 2.5 µL of 200 µM gRNA-21p-T94, and 5 µL of 200 µM tracRNA were mixed in the same microtube, and heated at 95°C for 3 minutes. After the heating, the temperature was decreased to room temperature. Then, the same technique as that of the CRISPR-Cas9-RNP electroporation described in Example 2 was performed. The target sequence of each gRNA is as follows.
gRNA-21p-TeloT6: 5'-AGACTGTAGCCTTGACCCAG-3' (SEQ ID NO: 15) + (AGG)
gRNA-21p-T94: 5'-CTGTGGACGTATTAACTTAC-3' (SEQ ID NO: 16) + (TGG)

Subsequently, the human iPS cells into which RNP was introduced were cultured for 2 days (48 hours) and 1 week, genomic DNAs were then extracted, and amplified by a PCR method using the genomes as templates, and using a forward primer Re21p-T94R (5'-ACCATTAGAGGACTCTGTCAGTG-3' (SEQ ID NO: 18)) specific for the short-arm of human chromosome 21 in the vicinity of the centromere and a reverse primer Chr21p-arm telo F7 (5'-TGTGCAACCAGCTTTGGAAC-3' (SEQ ID NO:17)) specific for the short-arm of human chromosome 21 in the vicinity of the telomere. The amounts of the template DNAs used per reaction in the PCR amplification were set at 100 ng, 10 ng, and 1 ng. Reaction was performed using KOD One^{(R)} PCR Master Mix manufactured by TOYOBO CO., LTD. as DNA polymerase, and the conditions of denaturing at 98°C for 10 seconds, annealing at 65°C for 5 seconds, elongation at 68°C for 5 seconds, and for 35 cycles. The amplified products were electrophoresed with a 2% agarose gel, then stained with ethidium bromide, to detect. The names and sequences of the primers are described as follows.
Chr21p-arm telo F7: 5'-TGTGCAACCAGCTTTGGAAC-3' (SEQ ID NO: 17)
Re21p-T94R: 5'-ACCATTAGAGGACTCTGTCAGTG-3' (SEQ ID NO: 18)

For the genomic DNAs after 1 week, the expected amplified products (about 680 bp) at 100 ng and 10 ng were detected (Figure 19). The diagram in the upper section shows the analysis results of the 201B7 cells which were disomic. The diagram in the lower section shows the analysis results of the 2767-4 cells which were trisomic. A DNA ladder marker, Gene ladder wide 2, was set as lane 1 from the left. Lanes 2 to lane 4 (After 48 H; 100 ng, 10 ng, and 1 ng) show the results of analysis using genomic DNAs collected 48 hours after the introduction of a combination of gRNAs for eliminating the short-arm of chromosome 21. The obtained PCR results showed that specific sequences generated when the short-arm of chromosome 21 was eliminated as intended (amplified product specific for eliminating short-arm of chromosome 21) appeared 48 hours after the introduction of RNP in both the 201B7 cells and 2767-4 cells when the amount of template DNA used was 100 ng. In the disomic 201B7 cells, sequences specific for chromosome 21 with the short-arm being eliminated were not detected when the amounts of template DNA used were 10 ng and 1 ng (upper section). In contrast, in the trisomic 2767-4 cells, a sequence specific for chromosome 21 with the short-arm being eliminated was detected even when the amount of template DNA used was 10 ng (lower section). This suggested that the short-arm of chromosome 21 was eliminated at a frequency of one or more cells in about 1500 cells (equivalent to 10 ng of template DNA) in the trisomic 2767-4 cells.

Lane 5 to lane 7 show the results of analysis using genomic DNAs collected 1 week after the introduction of a combination of gRNAs for eliminating the short-arm of chromosome 21 (after 1 week; 100 ng, 10 ng, and 1 ng). In the disomic 201B7 cells, an amplified product specific for chromosome 21 with the short-arm being eliminated was detected only when the amount of template DNA used was 100 ng (upper section). This suggested that the short-arm of chromosome 21 was eliminated at a frequency of one or more cells (0.0067%) out of about 15000 cells (equivalent to 100 ng of template DNA). In contrast, in the 2767-4 cells, amplified products specific for chromosome 21 with the short-arm being eliminated were detected when the amounts of template DNA used were 100 ng and 10 ng (lower section). This suggested that in the trisomic 2767-4 cells, the short-arm of chromosome 21 was eliminated at a frequency of one or more cells (0.067%) out of about 1500 cells (equivalent to 10 ng of template DNA). This suggested that the efficiency of elimination of the short-arm of chromosome 21 in the case of using the trisomic cell strain was 10 times compared to the case of using the human iPS cell strain 201B7.

### Industrial Applicability

The present invention provides a method for producing a human cell retaining a human artificial chromosome vector without nonhuman cell component, and is therefore expected to be a useful technique for, e.g., producing a cell pharmaceutical product that is safe for regenerative medicine or gene therapy.

### Free Text of Sequence Listing

SEQ ID NOs: 7 to 14: Primer
SEQ ID NOs: 15 and 16: Primer

All publications, patents, and patent applications cited herein are incorporated herein by reference in their entirety.

## Claims

1. A method for producing a human cell comprising a human artificial chromosome vector comprising: substantially eliminating endogenous genes of long-arm and short-arm in a disomic human cell containing a pair of the homologous human chromosomes or a trisomic human cell containing trisomy of the homologous human chromosomes, from one of two chromosomes of the disomy, or one or two of three chromosomes of the trisomy, thereby producing a cell population containing a human cell containing a human artificial chromosome vector comprising long-arm moiety and short-arm moiety substantially containing no endogenous gene, a human centromere, and a telomere; and collecting the human cell containing the human artificial chromosome vector from the cell population.

2. The method according to claim 1, wherein the collected human cell comprises two copies of a gene for haplo-insufficiency when the disomic human cell or the trisomic human cell comprises haplo-insufficiency.

3. The method according to claim 1 or 2, further comprising inserting a sequence for site-directed DNA insertion into the human artificial chromosome vector.

4. The method according to any one of claims 1 to 3, further comprising inserting an exogenous gene or DNA into the human artificial chromosome vector.

5. The method according to any one of claims 1 to 4, wherein the trisomic human cell is a cell originated from a trisomic human patient or a trisomic cell artificially produced from a disomic cell.

6. The method according to any one of claims 1 to 5, wherein the human cell is a somatic cell, a progenitor cell, or a stem cell.

7. A human cell comprising a pair of homologous human chromosomes and one human artificial chromosome vector originated from a human chromosome homogenous thereto, wherein the human artificial chromosome vector comprises long-arm moiety and short-arm moiety containing substantially no endogenous gene, a human centromere, and a telomere.

8. A human cell comprising one human chromosome and one or two human artificial chromosome vectors originated from a human chromosome homogenous thereto, wherein the human artificial chromosome vector comprises long-arm moiety and short-arm moiety substantially containing no endogenous gene, a human centromere, and a telomere, and the cell comprises two copies of a gene for haplo-insufficiency.

9. The human cell according to claim 7 or 8, wherein the human cell is a human cell produced by the method according to any one of claims 1 to 6.

10. The human cell according to any one of claims 7 to 9, wherein the human artificial chromosome vector comprises a sequence for site-directed DNA insertion.

11. The human cell according to any one of claims 7 to 10, wherein the human artificial chromosome vector comprises an exogenous gene or DNA.

12. The human cell according to any one of claims 7 to 11, wherein the human cell is a somatic cell, a progenitor cell, or a stem cell.

13. The human cell according to claim 12, wherein the stem cell is an iPS cell or mesenchymal stem cell.
